# EUROPEAN PATENT APPLICATION

(11) **EP 4 471 021 A1**
(43) Date of publication of application: **04.12.2024**
(21) Application number: 23746174.4
(22) Date of filing: 18.01.2023
(51) Int. Cl.: C07D 401/02, C07D 209/08, C07D 211/00, A61K 31/475, A61K 31/445, A61P 13/12, A61P 9/00, A61P 19/02

(54) **SALT TYPE AND CRYSTAL FORM OF COMPLEMENT FACTOR B INHIBITOR, AND PREPARATION METHOD THEREFOR AND APPLICATION THEREOF**

(30) Priority: 26.01.2022 CN 202210096073; 09.09.2022 CN 202211104894
(71) Applicant: Shanghai Meiyue Biotech Development Co., Ltd., Shanghai 200120 (CN)
(72) Inventor: LUAN, Linbo, Shanghai 200120 (CN); TIAN, Yong, Shanghai 200120 (CN); YAO, Yuanshan, Shanghai 200120 (CN); CHEN, Yongkai, Shanghai 200120 (CN); WANG, Chaodong, Shanghai 200120 (CN)
(74) Representative: Gille Hrabal Partnerschaftsgesellschaft mbB Patentanwälte
(86) International application number: PCT/CN2023/072834
(87) International publication number: WO 2023/143293

(57) **Abstract**

A pharmaceutically acceptable salt type and crystal form of a compound as represented by the following formula (I), and a preparation method of the compound and an application thereof.

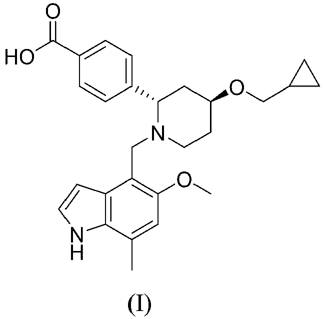

## Description

The present disclosure claims priority to the prior application with the patent application No. 202210096073.X entitled "SALT TYPE AND CRYSTAL FORM OF COMPLEMENT FACTOR B INHIBITOR, AND PREPARATION METHOD THEREFOR AND APPLICATION THEREOF" filed to China National Intellectual Property Administration on January 26, 2022 and the prior application with the patent application No. 202211104894.X entitled "SALT TYPE AND CRYSTAL FORM OF COMPLEMENT FACTOR B INHIBITOR, AND PREPARATION METHOD THEREFOR AND APPLICATION THEREOF" filed to China National Intellectual Property Administration on September 9, 2022, the contents of which are incorporated herein by reference in their entirety.

### TECHNICAL FIELD

The present disclosure belongs to the field of pharmaceuticals, and particularly relates to a salt form and a crystal form of a complement factor B inhibitor, a preparation method therefor, and use thereof.

### BACKGROUND

Complements are a class of soluble pattern recognition molecules in the immune system that can perform multiple effector functions. Under natural conditions, complement components are present as inactive zymogens, which can be broken down through a variety of specific and non-specific immunological mechanisms to produce large and small active fragments. The large fragments generally reside on the surface of pathogens or cells and lyse the latter or accelerate their clearance. The small fragments leave the cell surface and mediate multiple inflammatory responses. Complement activation consists of a process closely followed by another, and thus a cascade of reactions of complement activation form. Three primary complement activation pathways are known at present: the classical pathway, the lectin pathway, and the alternative pathway. Although the three complement activation pathways are started through different mechanisms and are activated in different orders, they share a common terminal pathway. The activation of the alternative pathway is independent of antigen-antibody complexes, and generally C3b deposited on the cell surface binds to factor B to be in such a state that it is easily decomposed by factor D in serum. In this process, factor B is decomposed into Ba and Bb. Then C3b and Bb form a complex as the C3 convertase C3bBb in the alternative pathway. In this process, complement factor B plays an early and leading role in the activation of the alternative pathway of the complement cascade. In this case, C3b is both a product of C3 being decomposed by the C3 convertase and a component of C3 convertase in the alternative pathway. As a result, there forms a feedback amplification mechanism of the interplay between the classical pathway and the alternative pathway. The current research reveals that many diseases such as blood, autoimmune, inflammatory and neurodegeneration diseases are associated with complement system dysfunction.

Paroxysmal nocturnal hemoglobinuria (PNH) is a chronic disease that causes constant hemolysis. It is a non-malignant clonal disease caused by acquired somatic mutation in the PIG-A gene of one or more hematopoietic stem cells, a very rare disease of the blood (Medicine (Baltimore) 1997, 76(2): 63-93). The course of the disease may manifest itself as various degrees of hemolytic exacerbation (paroxysmal), chronic or recurring episodes of acute intravascular hemolysis or subsequent venous/arterial thrombosis, which finally leads to progressive end-stage organ damage and death. In most patients, the disease is often atypical, insidious and persistent, and varies in severity.

There are more than ten kinds of proteins on the red cell surface that inhibit the activation of the complement pathways. They are all anchored to the cell membrane by glycosylated phosphatidylinositol (GPI) and thus are known collectively as GPI-anchored protein (AP). It is now believed that in the pathogenesis of PNH, hematopoietic stem cells are mutated first under certain conditions and glycosylphosphatidylinositol-deficient PNH clones are produced; then because of some factors (immune factors are mostly believed now to be the cause), hematopoietic impairment or hematopoietic failure is caused, and PNH clones gain an advantage in proliferation over normal clones. The multiple antigens to which GPI is linked also contribute to the complexity of the interpretation of PNH cell biological behaviors. C3 convertase decay accelerating factor CD55 and membrane attack complex (MAC) inhibitor CD59, the most important proteins that inhibit complement pathway activation, are closely related to PNH in pathogenesis, clinical manifestations, diagnosis and treatment (Frontiers in Immunology 2019, 10, 1157). CD59 can prevent C9 from being incorporated into C5b-8 complex and thus the formation of membrane attack units, thereby achieving the inhibition of end-stage attack responses of complements. It is now believed that the typical manifestations of PNH-intravascular hemolysis and thrombosis-are due to CD59 deficiency. Congenital CD59 deficiency patients are reported to exhibit numerous typical symptoms of PNH, such as intravascular hemolysis, hemoglobinuria and venous thrombosis and the like. In PNH patients, CD59 is unable to bind to the cell membrane of red blood cells due to GPI synthesis defects and thus loses its function of inhibiting complement pathway activation. Therefore, the complement pathways are abnormally activated and red blood cells are attacked, leading to various clinical manifestations such as intravascular hemolysis, hemoglobinuria and smooth muscle dysfunction and the like. At present, there is no other effective clinical cure for PNH except that it can be cured by reconstitution of normal hematopoietic function through hematopoietic stem cell transplantation. As hematopoietic stem cell transplantation involves an element of risk and PNH is a benign clonal disease, controlling hemolysis episodes remains a major strategy for the clinical treatment of this disease. At present, only eculizumab is approved for the treatment of PNH. However, many patients still experience anemia after being treated with eculizumab, and constant blood transfusion remains necessary for many of them. Moreover, eculizumab has to be intravenously injected when administered. Therefore, the development of novel inhibitors of the complement pathways is of great significance for the treatment of PNH.

IgAN is the most common primary glomerulonephritis. The disease is characterized by IgA deposition in the mesangial region indicated by immunofluorescence. The disease has diverse clinical manifestations, and usually manifests itself as recurrent microscopic or macroscopic hematuria. Available evidence suggests that the occurrence of IgAN is associated with congenital or acquired immune dysregulation. Due to irritation to the respiratory tract or the digestive tract caused by viruses, bacteria and food proteins and the like, mucosal IgA1 synthesis is increased, or IgA1-containing immune complexes are deposited in the mesangial region, thereby activating the alternative complement pathway and causing glomerular injury. Human IgA molecules are classified into 2 subtypes: IgA1 and IgA2. IgA1 is the major form (about 85%) of blood circulation in healthy individuals. It is also the major component of the deposition in the mesangial region in IgAN patients. IgA molecules can be present in monomeric form and in polymeric form. The IgA1 molecule contains a unique heavy chain hinge region between the first and second constant regions that can serve as a domain at the connection site for O-linked glycan groups. In recent years, it has been found that IgA molecules deposited in the serum and mesangial region of IgAN patients are mainly glycosylation-deficient IgA1(gd-IgA1). The abnormal increased production of gd-IgA1 is now believed to be the start of the pathogenesis of IgAN.

Complement C3 deposition occurs in the mesangial region of more than 90% of IgAN patients. Co-deposition of properdin, IgA and C3 occurs in kidney tissue of 75% to 100% of IgAN patients. Co-deposition of complement factors H, IgA and C3 occurs in kidney tissue of 30% to 90% of IgAN patients. In addition to deposition in kidney tissue, some studies have also revealed that the marker level of the alternative complement pathway in plasma of IgAN patients is also associated with the activity of IgAN (J Nephrol 2013, 26(4): 708-715). A study has confirmed that C3a in kidney tissue and urine and the C3a receptor in kidney tissue are significantly associated with the activity and severity of renal injury (J clin Immunol 2014, 34(2): 224-232). Other studies have confirmed that IgA is able to activate the alternative complement pathway *in vitro.* In this process, the abnormality in the IgA hinge region does not play a decisive role-rather, the formation of IgA multimer is a critical step (Eur J Immunol 1987, 17(3): 321-326). Complement C3 deposition in the glomerular mesangial region has now become a marker that assists in diagnosis of IgAN. In a study, 163 IgAN patients were subjected to immunofluorescence assays for C3c and C3d in kidney tissue. The results showed that IgAN patients in which the intensity of C3c deposition was stronger than that of C3d deposition had lower glomerular filtration rates, higher incidence of hyperplasia in the intraglomerular capillaries, and severer hematuria, which suggested that glomerular deposition of C3c was associated with the active pathological changes of IgAN (Am J Nephrol. 2000, 20(2): 122-128). At present, there is no specific drug for treating IgAN in clinical practice. General drugs such as renin-angiotensin inhibitors (ACEI or ARB), glucocorticoids and various immunosuppressive agents and the like are mainly used. In addition, the safety of such drugs is also a non-negligible topic. For example, although glucocorticoids can ameliorate proteinuria, STOP-IgAN tests and TESTING-I tests have clearly confirmed the potential side effects of glucocorticoids (IgA nephropathy 2019, 95, 4, 750-756).

Arthritis is a common chronic disease that is caused by inflammation, infection, degeneration, trauma or other factors and clinically manifests itself in red, swollen, hot, painful, dysfunctional and deformed joints. It often causes acute pain, decreased range of motion and deformity in people. Severe arthritis can cause disability, affecting the quality of life for patients. It was found that the serum of K/BxN mice could not induce arthritis in mice deficient in complement factor B but induce arthritis disease in wild-type mice (Immunity, 2002, 16, 157-168). This suggests that the complement system plays an important pathogenic role in the K/BxN mouse serum-induced arthritis model, and that complement factor B is a potential target for the treatment of arthritis.

Other diseases associated with the complement cascade include membranous nephropathy (MN), C3 glomerulonephritis (C3G), age-related macular degeneration (AMD), geographic atrophy (GA), atypical hemolytic uremic syndrome (aHUS), hemolytic uremic syndrome (HUS), hemodialysis complications, hemolytic anemia or hemodialysis, neuromyelitis optica (NMO), liver-related inflammations, inflammatory bowel disease, dermatomyositis and amyotrophic lateral sclerosis, myasthenia gravis (MG), respiratory diseases and cardiovascular diseases and the like.

At present, there is no small-molecule drugs of complement factor B inhibitors for clinical treatment. Currently known projects and those under development include: an oligonucleotide drug developed by IONIS Pharmaceuticals Inc. that is used as a complement factor B (CFB)-specific inhibitor for the treatment, prevention or alleviation of diseases associated with the dysregulation of the alternative complement pathway (WO2015038939). Small-molecule complement factor B inhibitors developed by Novartis AG Inc. are used for the treatment of diseases such as age-related macular degeneration (AMD) and the like (WO2013164802, WO2013192345, WO2014143638, WO2015009616, and WO2015066241) or for the treatment of diseases such as C3G and IgAN and the like (WO2019043609A1). A small-molecule complement factor B inhibitor developed by Achillion Pharmaceuticals Inc. is used for the treatment of diseases such as age-related macular degeneration (AMD) and the like (WO2018005552).

The inflammation and immune-related diseases are characterized by diversity and refractoriness. Eculizumab is the only drug available for PNH disease. However, the drug places a heavy burden on patients due to its prize. In addition, many patients still experience anemia after being treated with eculizumab, and constant blood transfusion remains necessary for many of them. Moreover, eculizumab has to be intravenously injected when administered. At present, there is no specific drug for the treatment of some diseases, such as IgAN and the like. There is an unmet clinical need in these areas. New small-molecule drugs need to be developed for medical treatment.

Therefore, there is a need to develop a pharmaceutically acceptable active ingredient with high efficacy, low toxicity and/or a long-acting effect to improve the technical problems described above.

### SUMMARY

In order to improve the technical problems described above, the present disclosure provides a pharmaceutically acceptable salt of a compound of formula I: 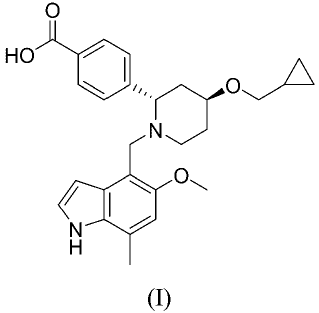 wherein the pharmaceutically acceptable salt is a salt formed by the compound of formula I and an acid or a base, and is preferably selected from a salt formed by the compound of formula I and an acid.

According to an embodiment of the present disclosure, the acid may be selected from an inorganic acid or an organic acid, e.g., hydrochloric acid, hydrofluoric acid, hydrobromic acid, hydroiodic acid, sulfuric acid, pyrosulfuric acid, phosphoric acid, nitric acid, formic acid, acetic acid, acetoacetic acid, pyruvic acid, trifluoroacetic acid, propionic acid, butyric acid, caproic acid, enanthic acid, undecanoic acid, lauric acid, benzoic acid, salicylic acid, 2-(4-hydroxybenzoyl)benzoic acid, camphoric acid, cinnamic acid, cyclopentanepropionic acid, digluconic acid, 3-hydroxy-2-naphthoic acid, nicotinic acid, pamoic acid, pectinic acid, persulfuric acid, 3-phenylpropionic acid, picric acid, pivalic acid, 2-hydroxyethanesulfonic acid, itaconic acid, sulfamic acid, trifluoromethanesulfonic acid, dodecylsulfuric acid, ethanesulfonic acid, benzenesulfonic acid, *p*-toluenesulfonic acid, methanesulfonic acid, 2-naphthalenesulfonic acid, naphthalenedisulfonic acid, camphorsulfonic acid, citric acid, L-tartaric acid, stearic acid, lactic acid, oxalic acid, malonic acid, succinic acid, malic acid, adipic acid, alginic acid, maleic acid, fumaric acid, D-gluconic acid, mandelic acid, ascorbic acid, glucoheptonic acid, glycerophosphoric acid, aspartic acid, sulfosalicylic acid, hemisulfuric acid, or thiocyanic acid. As an example, the acid may be selected from one of hydrochloric acid, hydrobromic acid, sulfuric acid, phosphoric acid, nitric acid, methanesulfonic acid, *p*-toluenesulfonic acid, fumaric acid, maleic acid, citric acid, L-tartaric acid, oxalic acid, formic acid, acetic acid, trifluoroacetic acid, lauric acid, benzoic acid, and benzenesulfonic acid.

According to an embodiment of the present disclosure, the base may be selected from an inorganic base, e.g., an alkali metal hydroxide or an alkaline-earth metal hydroxide, preferably selected from sodium hydroxide or potassium hydroxide.

According to a preferred embodiment of the present disclosure, the pharmaceutically acceptable salt of the compound of formula I is selected from one of hydrochloride, sulfate, phosphate, methanesulfonate, p-toluenesulfonate, fumarate, maleate, citrate, L-tartrate, and oxalate.

According to a more preferred embodiment of the present disclosure, the pharmaceutically acceptable salt of the compound of formula I is a salt formed by the compound of formula I and hydrochloric acid, i.e., hydrochloride of the compound of formula I, preferably monohydrochloride of the compound of formula I.

According to an embodiment of the present disclosure, in the pharmaceutically acceptable salt of the compound of formula I, the compound of formula I and the acid or the base may be in a molar ratio independently selected from 1:1, 2:1, or 3:1, with the proviso that the ion of the compound of formula I and the ion of the acid or the base in the salt are in charge balance. For example, when the number of ionizable hydrogen atoms in the acid (e.g., hydrochloric acid, methanesulfonic acid, and *p*-toluenesulfonic acid) is 1, the compound of formula I and the acid are in a molar ratio of 1:1; when the number of ionizable hydrogen atoms in the acid (e.g., sulfuric acid, fumaric acid, maleic acid, citric acid, L-tartaric acid, and oxalic acid) is 2, the compound of formula I and the acid may be in a molar ratio of 1:1 or 2:1; when the number of ionizable hydrogen atoms in the acid (e.g., phosphoric acid) is 3, the compound of formula I and the acid are in a molar ratio of 1:1, 2:1, or 3:1.

The present disclosure further provides a preparation method for the pharmaceutically acceptable salt of the compound of formula I, comprising: reacting the compound of formula I with the acid or the base to give the pharmaceutically acceptable salt of the compound of formula I.

According to an embodiment of the present disclosure, the preparation method comprises: reacting the compound of formula I with the acid or the base in a solvent to give the pharmaceutically acceptable salt of the compound of formula I.

According to an embodiment of the present disclosure, the acid or the base, independently of each other, has the definitions described above.

According to an embodiment of the present disclosure, the solvent may be selected from an alcohol, a ketone, an ester, an ether, a combination of two or more of the solvents, or a mixture of the solvent described above with water or a mixture of the combination described above with water.

According to an embodiment of the present disclosure, the alcohol may be selected from an alcohol having 1 to 8 carbon atoms, e.g., methanol, ethanol, *n*-propanol, isopropanol, *n*-butanol, neopentyl alcohol, or a combination of two or more thereof; the ketone may be selected from a ketone having 3 to 10 carbon atoms, e.g., acetone, butanone, pentanone, methyl ethyl ketone, 4-methyl-2-pentanone, or a combination of two or more thereof; the ester may be selected from an organic carboxylic ester, e.g., methyl formate, ethyl acetate, isobutyl formate, isopropyl acetate, or a combination of two or more thereof; the ether may be a linear or branched alkyl ether or a cyclic ether compound, e.g., methyl *tert*-butyl ether, tetrahydrofuran, 2-methyl-tetrahydrofuran, or a combination of two or more thereof.

According to an embodiment of the present disclosure, the compound of formula I and the acid or the base may be in a molar ratio of 1:0.8 to 1:1.5, preferably 1:0.9 to 1:1.3, and more preferably 1:1.0 to 1:1.1.

According to an embodiment of the present disclosure, in the preparation method, the reaction temperature may be selected within a relatively wide range, e.g., 20 °C to 80 °C, preferably 30 °C to 60 °C.

According to an embodiment of the present disclosure, the preparation method further comprises a step of filtering and/or drying after the reaction is completed to give the pharmaceutically acceptable salt of the compound of formula I.

According to an embodiment of the present disclosure, in the preparation method, the drying temperature may be selected within a relatively wide range, e.g., 20 °C to 80 °C, preferably 30 °C to 60 °C.

According to an embodiment of the present disclosure, in the preparation method, the drying pressure may be 0-20 KPa, preferably 0-10 KPa, and more preferably 5-10 KPa.

The present disclosure further provides a crystal, preferably a single crystal, of the monohydrochloride of the compound of formula I. The single crystal has the following unit cell parameters:
an orthorhombic crystal system with a space group of *P*2₁2₁2₁;
*a* = 9.4704 (18) Å;
*b* = 15.324 (4) Å;
*c* = 17.437 (4) Å;
*V =* 2530.5 (10) Å³;
*Z* = 4.

The present disclosure further provides a preparation method for the crystal of the monohydrochloride of the compound of formula I, particularly the single crystal thereof, which comprises: dissolving the monohydrochloride of the compound of formula I in a solvent A, and then placing the mixture in an atmosphere of a solvent B for diffusion.

The solvent A may be an alcohol solvent, e.g., a combination of two or more of methanol, ethanol, and the like.

The solvent B may be an ester solvent, an ether solvent, or a combination of two or more thereof. The ester solvent may be selected from an organic carboxylic ester, e.g., methyl formate, ethyl acetate, isobutyl formate, isopropyl acetate, or a combination of two or more thereof; the ether solvent may be a linear or branched alkyl ether, a cyclic ether compound, or a combination of two or more thereof, e.g., methyl *tert*-butyl ether, tetrahydrofuran, 2-methyl-tetrahydrofuran, or a combination of two or more thereof.

The present disclosure further provides a crystal form of the monohydrochloride of the compound of formula I, which is selected from crystal form A, crystal form B, crystal form C, crystal form D, or crystal form E described below.

The crystal form A of the monohydrochloride of the compound of formula I has characteristic peaks at 2θ angles of 9.66±0.20°, 16.08±0.20°, and 23.46±0.20° by X-ray powder diffraction using Cu-Kα radiation.

Preferably, the crystal form A has characteristic peaks at 2θ angles of 9.66±0.20°, 16.08±0.20°, 18.10±0.20°, 21.30±0.20°, and 21.68±0.20° by X-ray powder diffraction using Cu-Kα radiation.

Preferably, the crystal form A has characteristic peaks at 2θ angles of 9.66±0.20°, 11.62±0.20°, 16.08±0.20°, 18.10±0.20°, 21.30±0.20°, 21.68±0.20°, 23.40±0.20°, and 25.42±0.20° by X-ray powder diffraction using Cu-Kα radiation.

Preferably, the crystal form A has characteristic peaks at 2θ angles of 9.66±0.20°, 11.62±0.20°, 16.08±0.20°, 16.84±0.20°, 18.10±0.20°, 19.64±0.20°, 21.30±0.20°, 21.68±0.20°, 23.40±0.20°, 24.96±0.20°, and 25.42±0.20° by X-ray powder diffraction using Cu-Kα radiation.

Preferably, the crystal form A has characteristic peaks at 2θ angles as shown in Table 1 by X-ray powder diffraction using Cu-Kα radiation, wherein the 2θ angles have an error range of ±0.20°:

**Table 1. XRPD analysis data for crystal form A**

| Peak number | 2θ [°] | Relative intensity (%) | Peak number | 2θ [°] | Relative intensity (%) |
|---|---|---|---|---|---|
| 1 | 8.02 | 19.6 | 16 | 26.78 | 11.9 |
| 2 | 9.66 | 100 | 17 | 27.66 | 25.6 |
| 3 | 11.62 | 38.2 | 18 | 28.44 | 7.2 |
| 4 | 12.78 | 13.9 | 19 | 28.70 | 7.7 |
| 5 | 14.32 | 13.1 | 20 | 29.04 | 9.5 |
| 6 | 16.08 | 84.5 | 21 | 29.70 | 13.9 |
| 7 | 16.84 | 24 | 22 | 30.14 | 14.6 |
| 8 | 18.10 | 64.7 | 23 | 32.38 | 10.9 |
| 9 | 19.64 | 32 | 24 | 33.82 | 6.4 |
| 10 | 21.30 | 53.5 | 25 | 35.41 | 7.3 |
| 11 | 21.68 | 42.8 | 26 | 36.89 | 5.4 |
| 12 | 23.46 | 93.7 | 27 | 37.92 | 9.3 |
| 13 | 24.96 | 19 | 28 | 38.14 | 8.7 |
| 14 | 25.42 | 37.6 | 29 | 39.58 | 5 |
| 15 | 26.10 | 11.7 | | | |

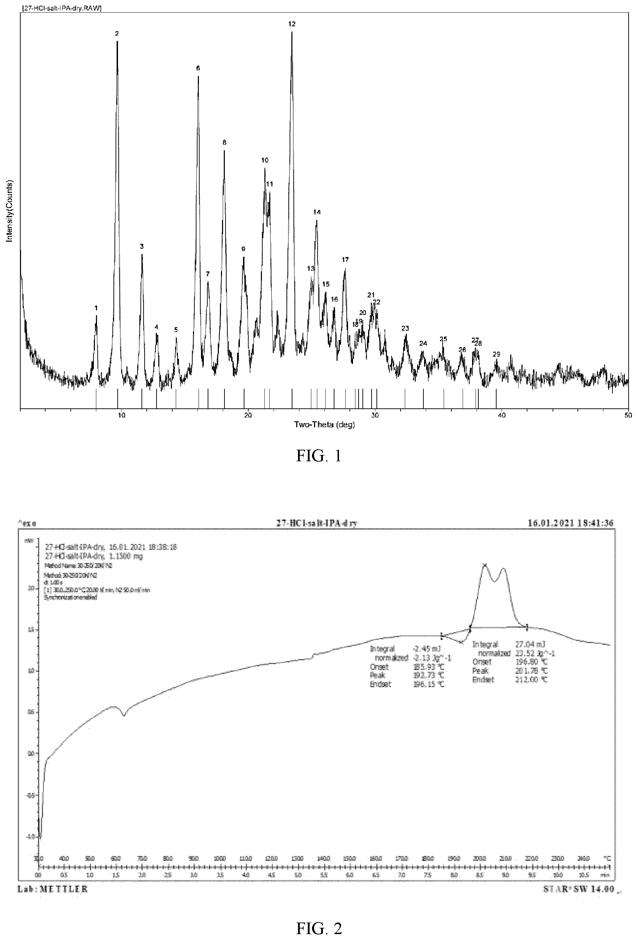

Preferably, the crystal form A has an X-ray powder diffraction pattern substantially as shown in FIG. 1. According to an embodiment of the present disclosure, the crystal form A is an anhydrate of the monohydrochloride of the compound of formula I.

According to an embodiment of the present disclosure, differential scanning calorimetry (DSC) analysis of the crystal form A shows a first endothermic peak at a peak temperature raised to about 192.73 °C and a first exothermic peak at a peak temperature of about 201.78 °C.

Preferably, the crystal form A has a DSC profile substantially as shown in FIG. 2.

According to an embodiment of the present disclosure, thermogravimetric analysis (TGA) of the crystal form A shows a weight loss of about 1.41% from 90 °C to 180 °C.

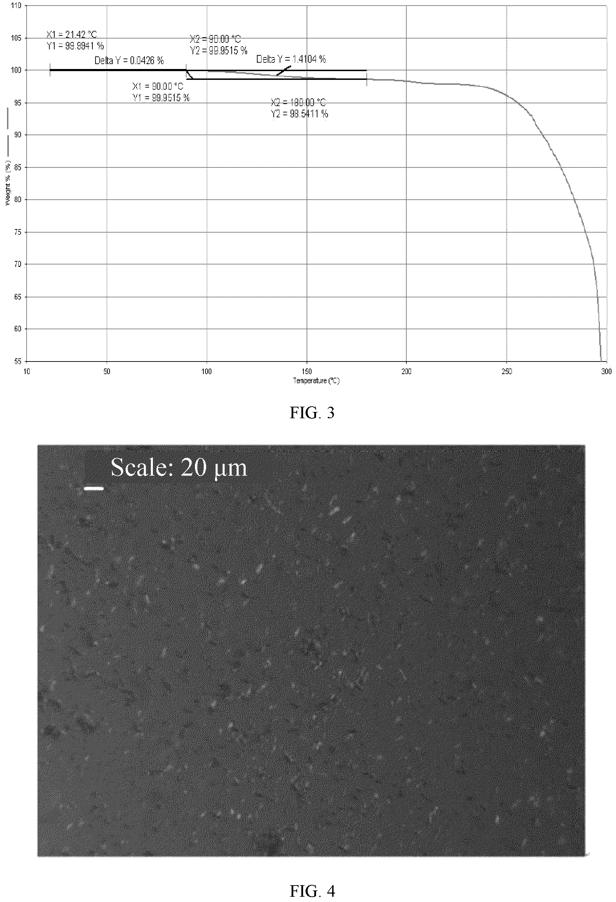

Preferably, the crystal form A has a TGA profile substantially as shown in FIG. 3.

According to an embodiment of the present disclosure, the crystal form A is a crystal with irregular morphology. Preferably, the crystal form A has a particle size of no more than 20 µm.

Preferably, the crystal form A has a PLM image substantially as shown in FIG. 4.

The crystal form B of the monohydrochloride of the compound of formula I has characteristic peaks at 2θ angles of 18.10±0.20°, 19.80±0.20°, and 22.10±0.20° by X-ray powder diffraction using Cu-Kα radiation.

Preferably, the crystal form B has characteristic peaks at 2θ angles of 9.48±0.20°, 15.44±0.20°, 18.10±0.20°, 19.80±0.20°, 22.10±0.20°, and 30.92±0.20° by X-ray powder diffraction using Cu-Kα radiation.

Preferably, the crystal form B has characteristic peaks at 2θ angles of 9.48±0.20°, 10.78±0.20°, 15.44±0.20°, 18.10±0.20°, 19.18±0.20°, 19.80±0.20°, 22.10±0.20°, and 30.92±0.20° by X-ray powder diffraction using Cu-Kα radiation.

Preferably, the crystal form B has characteristic peaks at 2θ angles as shown in Table 2 by X-ray powder diffraction using Cu-Kα radiation, wherein the 2θ angles have an error range of ±0.20°:

**Table 2. XRPD analysis data for crystal form B**

| Peak number | 2θ [°] | Relative intensity (%) | Peak number | 2θ [°] | Relative intensity (%) |
|---|---|---|---|---|---|
| 1 | 9.48 | 32.4 | 13 | 23.40 | 8.2 |
| 2 | 10.78 | 22.6 | 14 | 24.10 | 13.3 |
| 3 | 12.08 | 7.1 | 15 | 25.41 | 7.8 |
| 4 | 14.68 | 14.5 | 16 | 26.22 | 9 |
| 5 | 15.44 | 27.8 | 17 | 28.16 | 14.4 |
| 6 | 18.10 | 51.2 | 18 | 29.18 | 8 |
| 7 | 19.18 | 20.7 | 19 | 29.58 | 5.5 |
| 8 | 19.80 | 49.9 | 20 | 30.13 | 5.2 |
| 9 | 20.60 | 17.9 | 21 | 30.92 | 24.4 |
| 10 | 21.34 | 12.4 | 22 | 33.02 | 4.6 |
| 11 | 22.10 | 100 | 23 | 35.50 | 8.7 |
| 12 | 22.94 | 9.3 | 24 | 41.28 | 4.8 |

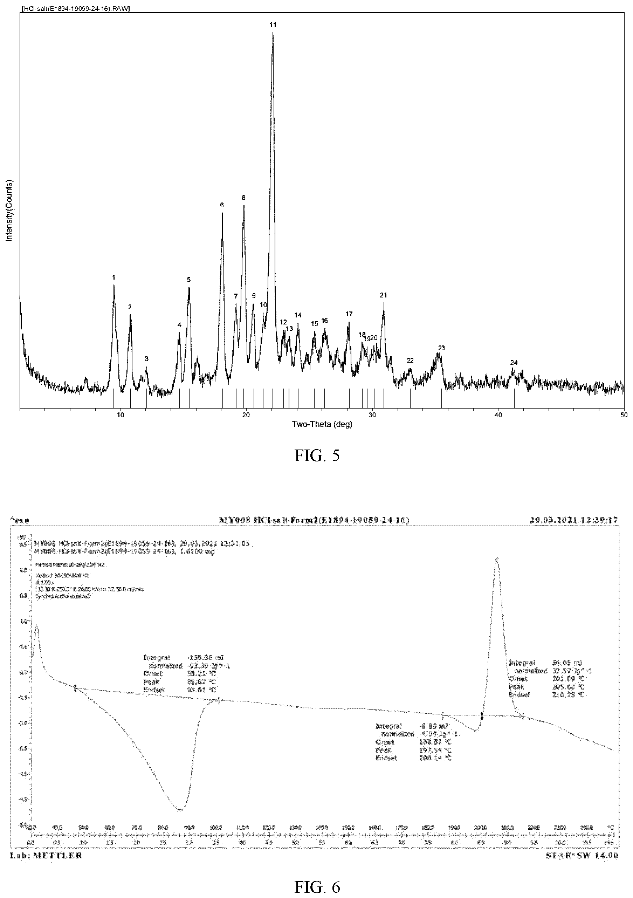

Preferably, the crystal form B has an X-ray powder diffraction pattern substantially as shown in FIG. 5. According to an embodiment of the present disclosure, the crystal form B is a hydrate of the monohydrochloride of the compound of formula I, such as a monohydrate of the monohydrochloride of the compound of formula I.

According to an embodiment of the present disclosure, differential scanning calorimetry (DSC) analysis of the crystal form B shows a first endothermic peak at a peak temperature raised to about 85.87 °C, a second endothermic peak at a peak temperature of about 197.54 °C, and a first exothermic peak at a peak temperature of about 205.68 °C.

Preferably, the crystal form B has a DSC profile substantially as shown in FIG. 6.

According to an embodiment of the present disclosure, thermogravimetric analysis (TGA) of the crystal form B shows a weight loss of about 3.42% from 21.49 °C to 120 °C and a weight loss of about 0.49% from 179.88 °C to 207.94 °C.

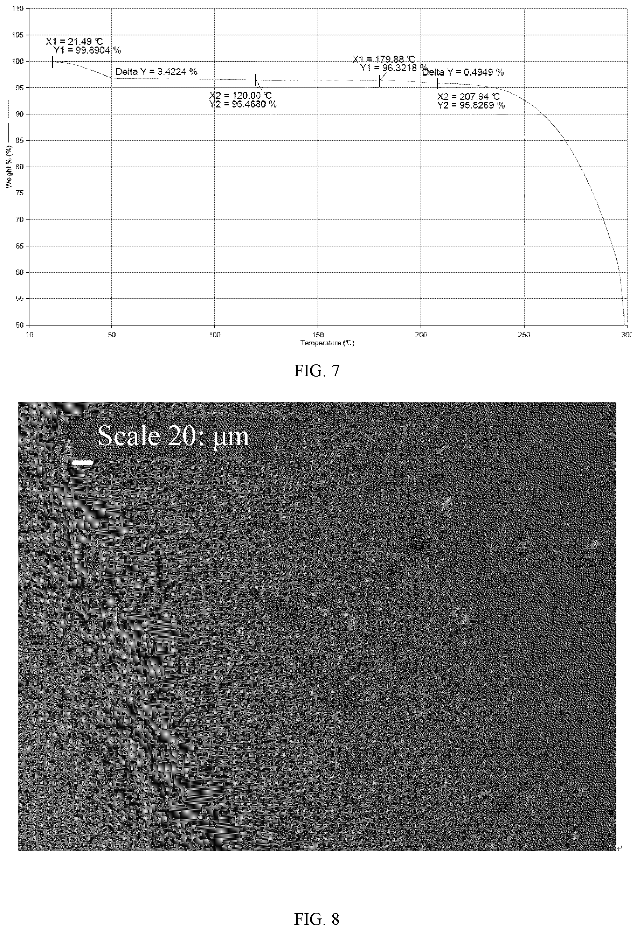

Preferably, the crystal form B has a TGA profile substantially as shown in FIG. 7.

According to an embodiment of the present disclosure, the crystal form B is a crystal with irregular morphology. Preferably, the crystal form B has a particle size of no more than 20 µm.

Preferably, the crystal form B has a PLM image substantially as shown in FIG. 8.

According to an embodiment of the present disclosure, the crystal form B is obtained from the crystal form A under high humidity conditions. The high humidity conditions are preferably 75%-95% relative humidity at 40 °C.

According to an embodiment of the present disclosure, the crystal form A is obtained from the crystal form B under drying conditions. The drying conditions are preferably drying in vacuum at 40 °C.

The crystal form C of the monohydrochloride of the compound of formula I has characteristic peaks at 2θ angles of 14.74±0.20°, 17.80±0.20°, 20.08±0.20°, and 21.98±0.20° by X-ray powder diffraction using Cu-Kα radiation.

Preferably, the crystal form C has characteristic peaks at 2θ angles of 14.74±0.20°, 17.80±0.20°, 19.58±0.20°, 20.08±0.20°, 21.98±0.20°, 22.94±0.20°, and 25.92±0.20° by X-ray powder diffraction using Cu-Kα radiation.

Preferably, the crystal form C has characteristic peaks at 2θ angles of 14.74±0.20°, 17.80±0.20°, 19.58±0.20°, 20.08±0.20°, 21.98±0.20°, 22.94±0.20°, 25.92±0.20°, and 33.48±0.20° by X-ray powder diffraction using Cu-Kα radiation.

Preferably, the crystal form C has characteristic peaks at 2θ angles as shown in Table 3 by X-ray powder diffraction using Cu-Kα radiation, wherein the 2θ angles have an error range of ±0.20°:

**Table 3. XRPD analysis data for crystal form C**

| Peak number | 2θ [°] | Relative intensity (%) | Peak number | 2θ [°] | Relative intensity (%) |
|---|---|---|---|---|---|
| 1 | 10.00 | 15.7 | 14 | 21.98 | 69.7 |
| 2 | 10.52 | 12.7 | 15 | 22.94 | 36.2 |
| 3 | 11.96 | 21 | 16 | 24.80 | 17.1 |
| 4 | 12.48 | 12.7 | 17 | 25.92 | 40.4 |
| 5 | 13.62 | 13.2 | 18 | 27.46 | 22.7 |
| 6 | 14.74 | 100 | 19 | 29.06 | 27.8 |
| 7 | 15.58 | 10.9 | 20 | 30.30 | 16.3 |
| 8 | 17.80 | 70.2 | 21 | 31.54 | 6.1 |
| 9 | 18.58 | 17.7 | 22 | 32.10 | 13.2 |
| 10 | 18.90 | 14.9 | 23 | 33.48 | 29.3 |
| 11 | 19.58 | 43.8 | 24 | 33.94 | 7.5 |
| 12 | 20.08 | 88.8 | 25 | 36.16 | 6.8 |
| 13 | 21.06 | 20 | 26 | 42.06 | 9.5 |

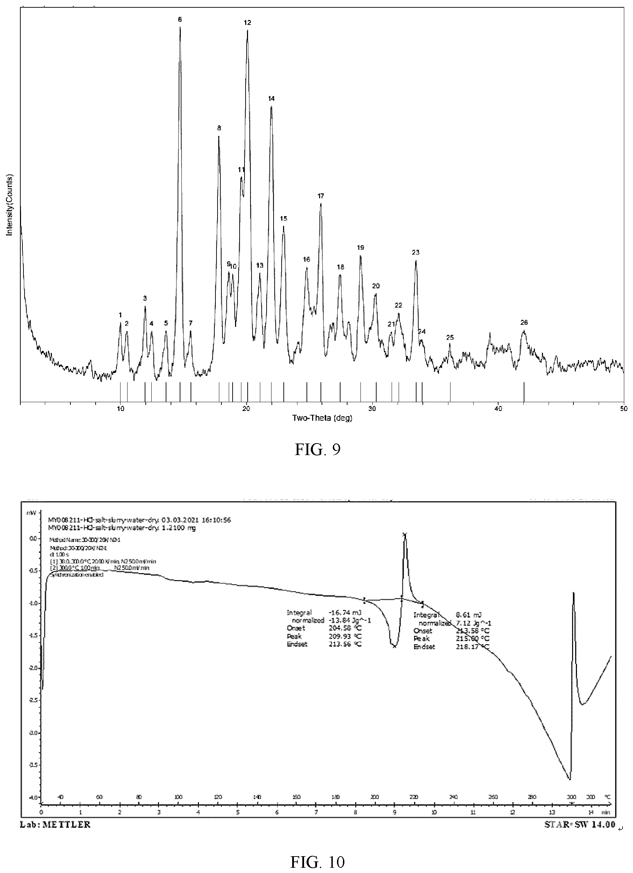

Preferably, the crystal form C has an X-ray powder diffraction pattern substantially as shown in FIG. 9.

According to an embodiment of the present disclosure, the crystal form C is an anhydrate of the monohydrochloride of the compound of formula I.

According to an embodiment of the present disclosure, differential scanning calorimetry (DSC) analysis of the crystal form C shows a first endothermic peak at a peak temperature raised to about 209.93 °C and a first exothermic peak at a peak temperature of about 215.80 °C.

Preferably, the crystal form C has a DSC profile substantially as shown in FIG. 10.

According to an embodiment of the present disclosure, thermogravimetric analysis (TGA) of the crystal form C shows a weight loss of about 0.29% from 21.62 °C to 120 °C and a weight loss of about 0.52% from 173.94 °C to 216.60 °C.

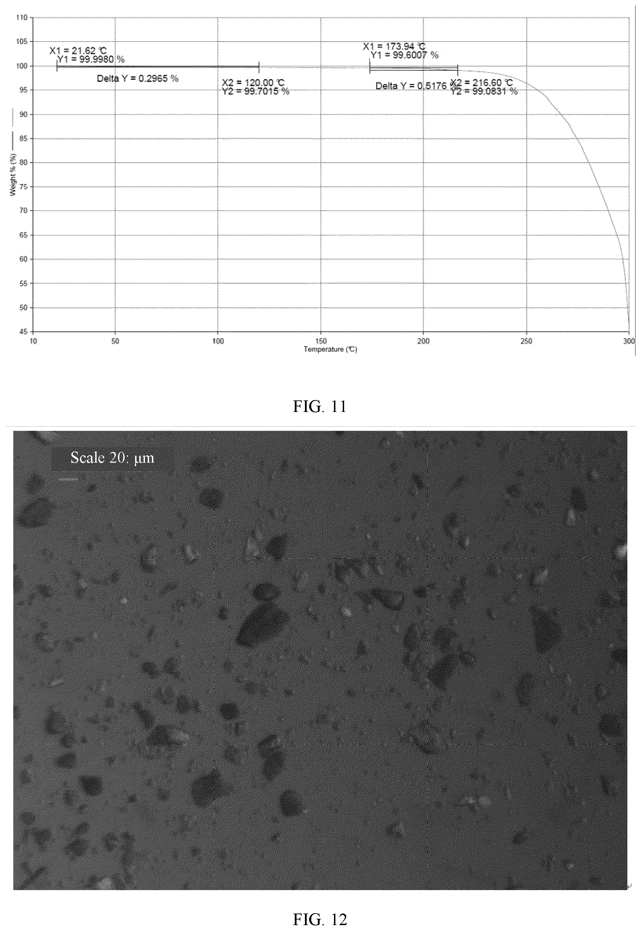

Preferably, the crystal form C has a TGA profile substantially as shown in FIG. 11.

According to an embodiment of the present disclosure, the crystal form C is a crystal with irregular morphology. Preferably, the crystal form C has a particle size of no more than 20 µm.

Preferably, the crystal form C has a PLM image substantially as shown in FIG. 12.

The crystal form D of the monohydrochloride of the compound of formula I has characteristic peaks at 2θ angles of 15.74±0.20°, 16.58±0.20°, 21.98±0.20°, and 23.82±0.20° by X-ray powder diffraction using Cu-Kα radiation.

Preferably, the crystal form D has characteristic peaks at 2θ angles of 10.16±0.20°, 11.90±0.20°, 15.74±0.20°, 16.58±0.20°, 19.22±0.20°, 20.24±0.20°, 21.98±0.20°, and 23.82±0.20° by X-ray powder diffraction using Cu-Kα radiation.

Preferably, the crystal form D has characteristic peaks at 2θ angles of 10.16±0.20°, 11.90±0.20°, 12.60±0.20°, 15.74±0.20°, 16.58±0.20°, 19.22±0.20°, 19.80±0.20°, 21.98±0.20°, 22.66±0.20°, 23.18±0.20°, 23.82±0.20°, 24.94±0.20°, 26.24±0.20°, 26.80±0.20°, and 27.50±0.20° by X-ray powder diffraction using Cu-Kα radiation.

Preferably, the crystal form D has characteristic peaks at 2θ angles as shown in Table 4 by X-ray powder diffraction using Cu-Kα radiation, wherein the 2θ angles have an error range of ±0.20°:

**Table 4. XRPD analysis data for crystal form D**

| Peak number | 2θ [°] | Relative intensity (%) | Peak number | 2θ [°] | Relative intensity (%) |
|---|---|---|---|---|---|
| 1 | 10.16 | 37.6 | 13 | 23.82 | 43.9 |
| 2 | 11.90 | 25.6 | 14 | 24.94 | 11.8 |
| 3 | 12.60 | 14.6 | 15 | 25.60 | 9.8 |
| 4 | 15.74 | 100 | 16 | 26.24 | 23.3 |
| 5 | 16.58 | 43.5 | 17 | 26.80 | 11.7 |
| 6 | 19.22 | 40.1 | 18 | 27.50 | 12.9 |
| 7 | 19.80 | 13.9 | 19 | 29.36 | 7 |
| 8 | 20.24 | 26.4 | 20 | 29.88 | 13.6 |
| 9 | 21.12 | 9.8 | 21 | 31.00 | 6.6 |
| 10 | 21.98 | 41.1 | 22 | 32.48 | 4.8 |
| 11 | 22.66 | 10.4 | 23 | 36.20 | 6.3 |
| 12 | 23.18 | 14.4 | 24 | 36.76 | 4.4 |

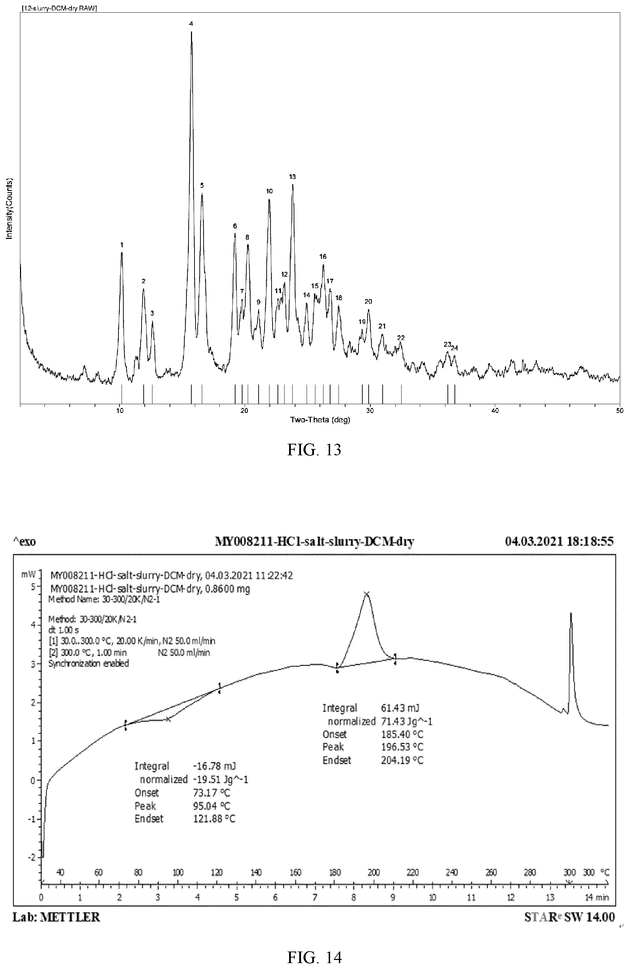

Preferably, the crystal form D has an X-ray powder diffraction pattern substantially as shown in FIG. 13. According to an embodiment of the present disclosure, the crystal form D is a solvate of the monohydrochloride of the compound of formula I, for example, a dichloromethane solvate of the monohydrochloride of the compound of formula I, such as a mono-dichloromethane solvate (also referred to as monodichloromethane solvate) of the monohydrochloride of the compound of formula I. According to an embodiment of the present disclosure, differential scanning calorimetry (DSC) analysis of the crystal form D shows a first exothermic peak at a peak temperature raised to about 196.53 °C.

Preferably, the crystal form D has a DSC profile substantially as shown in FIG. 14.

According to an embodiment of the present disclosure, thermogravimetric analysis (TGA) of the crystal form D shows a weight loss of about 6.31% from 22.07 °C to 120 °C.

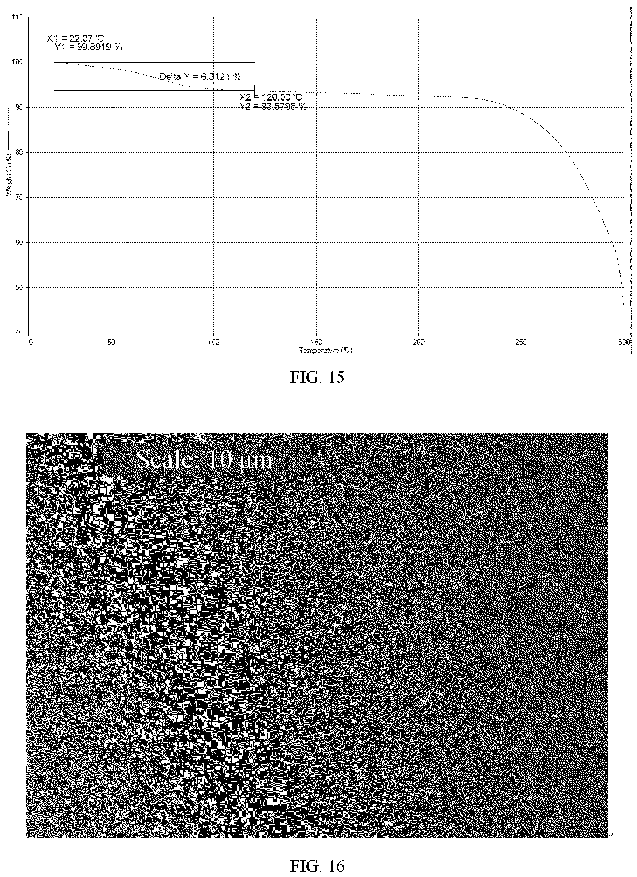

Preferably, the crystal form D has a TGA profile substantially as shown in FIG. 15.

According to an embodiment of the present disclosure, the crystal form D is a crystal with irregular morphology. Preferably, the crystal form C has a particle size of no more than 10 µm.

Preferably, the crystal form D has a PLM image substantially as shown in FIG. 16.

The crystal form E of the monohydrochloride of the compound of formula I has characteristic peaks at 2θ angles of 9.36±0.20°, 15.22±0.20°, 16.88±0.20°, and 22.10±0.20° by X-ray powder diffraction using Cu-Kα radiation.

Preferably, the crystal form E has characteristic peaks at 2θ angles of 7.20±0.20°, 9.36±0.20°, 15.22±0.20°, 16.88±0.20°, 21.10±0.20°, 22.10±0.20°, and 23.68±0.20° by X-ray powder diffraction using Cu-Kα radiation.

Preferably, the crystal form E has characteristic peaks at 2θ angles of 7.20±0.20°, 9.36±0.20°, 15.22±0.20°, 16.88±0.20°, 18.78±0.20°, 21.10±0.20°, 22.10±0.20°, 23.68±0.20°, 26.04±0.20°, and 27.86±0.20° by X-ray powder diffraction using Cu-Kα radiation.

Preferably, the crystal form E has characteristic peaks at 2θ angles as shown in Table 5 by X-ray powder diffraction using Cu-Kα radiation, wherein the 2θ angles have an error range of ±0.20°:

**Table 5. XRPD analysis data for crystal form E**

| Peak number | 2θ [°] | Relative intensity (%) | Peak number | 2θ [°] | Relative intensity (%) |
|---|---|---|---|---|---|
| 1 | 7.20 | 35.5 | 12 | 22.10 | 64.1 |
| 2 | 9.36 | 100 | 13 | 22.70 | 13.1 |
| 3 | 11.32 | 14.4 | 14 | 23.68 | 34.4 |
| 4 | 12.98 | 8 | 15 | 26.04 | 24.2 |
| 5 | 15.22 | 58.7 | 16 | 26.90 | 9.7 |
| 6 | 16.88 | 59 | 17 | 27.86 | 27.3 |
| 7 | 17.90 | 9.5 | 18 | 29.38 | 11.5 |
| 8 | 18.78 | 27.7 | 19 | 30.56 | 10 |
| 9 | 19.18 | 23.1 | 20 | 33.28 | 14.8 |
| 10 | 19.92 | 20 | 21 | 37.54 | 8.4 |
| 11 | 21.10 | 40.3 | | | |

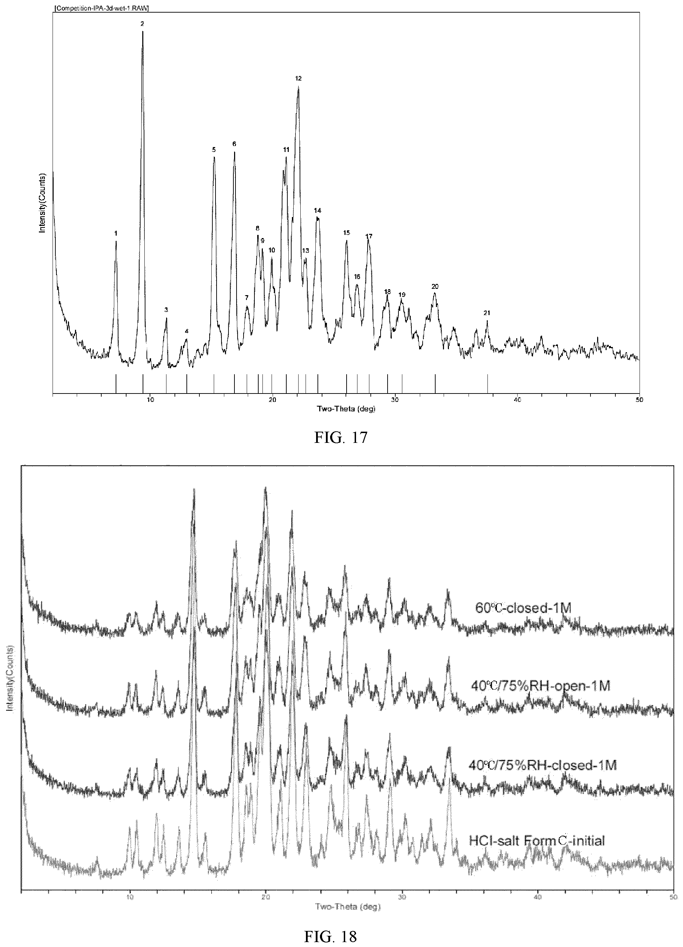

Preferably, the crystal form E has an X-ray powder diffraction pattern substantially as shown in FIG. 17.

According to an embodiment of the present disclosure, the crystal form E is a solvate of the monohydrochloride of the compound of formula I, such as a mono-isopropanol solvate of the monohydrochloride of the compound of formula I.

The present disclosure further provides preparation methods for the crystal forms of the monohydrochloride of the compound of formula I described above.

Preparation method 1 for the crystal form A comprises: dissolving the compound of formula I in an alcohol solvent, adding a solution of HCl in the alcohol solvent to form a salt, and then adding an n-alkane for crystallization to give the crystal form A.

The alcohol solvent is selected from ethanol and/or isopropanol, preferably isopropanol.

The n-alkane is selected from n-hexane and/or n-heptane, preferably n-heptane.

The compound of formula I, the alcohol solvent, and the n-alkane are in a mass-to-volume ratio of 1 g:(10-30) mL:(10-30) mL, preferably 1 g:(15-25) mL:(15-25) mL.

The solution of HCl in the alcohol solvent is at a concentration of 1-3 mol/L, such as 2 mol/L.

The heating temperature is 45-75 °C, preferably 48-60 °C.

Preparation method 2 for the crystal form A comprises: heating and stirring the monohydrochloride of the compound of formula I in an alcohol solvent and an *n*-alkane until it is completely dissolved, and then crystallizing to give the crystal form A.

The alcohol solvent is selected from ethanol and/or isopropanol, preferably isopropanol.

The n-alkane is selected from *n*-hexane and/or *n*-heptane, preferably *n*-heptane.

The monohydrochloride of the compound of formula I, the alcohol solvent, and the *n*-alkane are in a mass-to-volume ratio of 1 g:(10-30) mL:(10-30) mL, preferably 1 g:(15-25) mL:(15-25) mL, such as 1 g:20 mL:20 mL.

The heating temperature is 45-75 °C, preferably 48-60 °C.

According to an embodiment of the present disclosure, preparation method 1 or 2 for the crystal form A further comprises the steps of cooling, filtering, and drying.

According to a preferred embodiment of the present disclosure, the preparation method for the crystal form A comprises: dissolving the compound of formula I in isopropanol, adding a solution of HCl in isopropanol, mixing and dissolving, adding n-heptane, stirring, filtering, and drying to give the crystal form A.

According to a preferred embodiment of the present disclosure, the preparation method for the crystal form A comprises: adding the monohydrochloride of the compound of formula I to a mixed solvent of isopropanol and *n*-heptane, heating and stirring, cooling to normal temperature, filtering, and drying in vacuum to give the crystal form A.

The monohydrochloride of the compound of formula I, isopropanol, and *n*-heptane are in a mass-to-volume ratio of 1 g:(10-30) mL:(10-30) mL, such as 1 g:20 mL:20 mL.

The preparation method for the crystal form B comprises: putting the crystal form A under a high humidity condition to give the crystal form B.

According to an embodiment of the present disclosure, the high humidity condition has a temperature of 30-50 °C and a humidity of 60%-98%.

The high humidity condition is preferably 75%-95% humidity at 40 °C.

The preparation method for the crystal form C comprises: dissolving the compound of formula I in an alcohol solvent, adding a solution of HCl in the alcohol solvent to form a salt, and then adding an ether solvent or an ester solvent for crystallization to give the crystal form C.

According to an embodiment of the present disclosure, the preparation method for the crystal form C comprises: dissolving the compound of formula I in an alcohol solvent, adding a solution of HCl in the alcohol solvent, stirring, filtering, dropwise adding an ether solvent or an ester solvent to the filtrate, stirring, filtering, and drying to give the crystal form C.

The alcohol solvent is selected from methanol, ethanol, or isopropanol, preferably methanol.

The ether solvent is selected from methyl ether, diethyl ether, propyl ether, or methyl *tert*-butyl ether, preferably methyl *tert*-butyl ether.

The ester solvent is selected from ethyl acetate or isopropyl acetate.

The compound of formula I, the alcohol solvent, and the ether solvent or ester solvent are in a mass-to-volume ratio of 1 g:(2-8) mL:(20-40) mL, preferably 1 g:(3-6) mL:(20-30) mL, such as 1 g:4 mL:25 mL.

The solution of HCl in the alcohol solvent is at a concentration of 1-3 mol/L, such as 1.5-2.5 mol/L, and illustratively 1.8 mol/L; the compound of formula I and the solution of HCl in the alcohol solvent are in a mass ratio of 1 g:(0.5-1.5) g, such as 1 g:(0.8-1.2) g.

According to a preferred embodiment of the present disclosure, the preparation method for the crystal form C comprises: dissolving the compound of formula I in methanol, adding a solution of HCl in methanol, stirring, filtering, adding methyl *tert*-butyl ether to the filtrate, filtering, and drying to give the crystal form C.

The preparation method for the crystal form D comprises: suspending and stirring the monohydrochloride of the compound of formula I in a haloalkane at room temperature for crystallization to give the crystal form D.

According to an embodiment of the present disclosure, the preparation method for the crystal form D comprises: adding the monohydrochloride of the compound of formula I to a haloalkane, stirring, separating the resulting suspension, and drying the separated solid to give a solid, i.e., the crystal form D.

The haloalkane is selected from dichloromethane, trichloromethane, or tetrachloromethane, preferably dichloromethane.

The monohydrochloride of the compound of formula I and the haloalkane are in a mass-to-volume ratio of 1 g:(15-35) mL, preferably 1 g:(18-25) mL, such as 1 g:20 mL.

The separation is performed by known separation methods, preferably by centrifugation.

The drying is vacuum drying under reduced pressure under heating conditions, preferably vacuum drying under reduced pressure at 40 °C.

According to a preferred embodiment of the present disclosure, the preparation method for the crystal form D comprises: adding the monohydrochloride of the compound of formula I to dichloromethane, stirring at room temperature, and separating the solid to give the crystal form D.

The preparation method for the crystal form E comprises: suspending and stirring the monohydrochloride of the compound of formula I in an alcohol solvent at room temperature for crystallization to give the crystal form E.

According to an embodiment of the present disclosure, the monohydrochloride of the compound of formula I is added to an alcohol solvent and stirred, the resulting suspension is separated, and the separated solid is dried to give a solid, i.e., the crystal form E.

The alcohol solvent is selected from methanol, ethanol, or isopropanol, preferably isopropanol.

The monohydrochloride of the compound of formula I and the alcohol solvent are in a mass-to-volume ratio of 1 g:(15-35) mL, preferably 1 g:(18-25) mL, such as 1 g:20 mL.

The separation is performed by known separation methods, preferably by centrifugation.

The drying is vacuum drying under reduced pressure under heating conditions, preferably vacuum drying under reduced pressure at 40 °C.

According to a preferred embodiment of the present disclosure, the preparation method for the crystal form E comprises: adding the monohydrochloride of the compound of formula I to isopropanol, stirring at room temperature, and separating the solid to give the crystal form E.

The present disclosure further provides a pharmaceutical composition comprising at least one of the pharmaceutically acceptable salts of the compound of formula I (e.g., the hydrochloride, such as the crystal form A, crystal form B, crystal form C, crystal form D, and crystal form E of the hydrochloride), and optionally a pharmaceutically acceptable auxiliary material. Preferably, the pharmaceutical composition is in the form of a preparation.

The present disclosure further provides a preparation comprising at least one of the pharmaceutically acceptable salts of the compound of formula I, crystal form A, crystal form B, crystal form C, crystal form D, and crystal form E, and optionally a pharmaceutically acceptable auxiliary material.

The present disclosure further provides use of at least one of the pharmaceutically acceptable salts of the compound of formula I as described above (e.g., the hydrochloride, such as the crystal form A, crystal form B, crystal form C, crystal form D, and crystal form E of the hydrochloride) or the pharmaceutical composition for the manufacture of a medicament for preventing and/or treating a complement factor B-mediated disease or disorder.

According to an embodiment of the present disclosure, the complement factor B-mediated disease or disorder is selected from at least one of the following: paroxysmal nocturnal hemoglobinuria (PNH), primary glomerulonephritis (IgAN), membranous nephropathy (MN), C3 glomerulonephritis (C3G), age-related macular degeneration (AMD), geographic atrophy (GA), atypical hemolytic uremic syndrome (aHUS), hemolytic uremic syndrome (HUS), diabetic retinopathy (DR), hemodialysis complications, hemolytic anemia or hemodialysis, neuromyelitis optica (NMO), arthritis, rheumatoid arthritis, liver-related inflammations, dermatomyositis and amyotrophic lateral sclerosis, myasthenia gravis (MG), respiratory diseases and cardiovascular diseases, and the like.

The present disclosure further provides a method for preventing and/or treating a disease associated with a complement factor B inhibitor, comprising administering to an individual in need thereof a therapeutically effective amount of at least one of the pharmaceutically acceptable salts of the compound of formula I as described above (e.g., the hydrochloride, such as the crystal form A, crystal form B, crystal form C, crystal form D, and crystal form E of the hydrochloride) or the pharmaceutical composition.

According to an embodiment of the present disclosure, the disease or disorder associated with the complement factor B inhibitor is selected from at least one of the following: paroxysmal nocturnal hemoglobinuria (PNH), primary glomerulonephritis (IgAN), membranous nephropathy (MN), C3 glomerulonephritis (C3G), age-related macular degeneration (AMD), geographic atrophy (GA), atypical hemolytic uremic syndrome (aHUS), hemolytic uremic syndrome (HUS), diabetic retinopathy (DR), hemodialysis complications, hemolytic anemia or hemodialysis, neuromyelitis optica (NMO), arthritis, rheumatoid arthritis, liver-related inflammations, dermatomyositis and amyotrophic lateral sclerosis, myasthenia gravis (MG), respiratory diseases and cardiovascular diseases, and the like.

The treatment method of the present disclosure may comprise administering the pharmaceutically acceptable salts of the compound of formula I (e.g., the hydrochloride, such as the crystal form A, crystal form B, crystal form C, crystal form D, and crystal form E of the hydrochloride) or the pharmaceutical composition of the present disclosure alone, and administering one, two or more of the pharmaceutically acceptable salts of the compound of formula I (e.g., the hydrochloride, such as the crystal form A, crystal form B, crystal form C, crystal form D, and crystal form E of the hydrochloride) or the pharmaceutical composition of the present disclosure in combination with one, two or more other chemotherapeutic agents. Multiple drugs may be administered simultaneously or successively.

In the context of this specification, "or more", "or less", or "within" should be understood to include the number itself. As an example, "at least one" should be understood as "one, two or more". As another example, "more than two" should be understood as "two or more", such as "two, three, four or more".

### Beneficial Effects

The salts (particularly hydrochloride, phosphate, and maleate) of the compound of formula I of the present disclosure have high stability and high water solubility, and significantly enhance the absorption capacity during oral administration and improve the bioavailability. Moreover, the crystal forms of the hydrochloride of the compound of formula I of the present disclosure have high stability, good solubility, low hygroscopicity, and good pharmaceutical prospects. In addition, the preparation methods for the salts and the crystal forms of the compound of formula I of the present disclosure are convenient to operate and easy to control, have good reproducibility, mild reaction conditions and high product yield, and thus are beneficial to industrial production.

### BRIEF DESCRIPTION OF THE DRAWINGS

FIG. 1 is an XRPD pattern of crystal form A of monohydrochloride of the compound of formula I.
FIG. 2 is a DSC profile of the crystal form A of the monohydrochloride of the compound of formula I.
FIG. 3 is a TGA profile of the crystal form A of the monohydrochloride of the compound of formula I.
FIG. 4 is a PLM image of the crystal form A of the monohydrochloride of the compound of formula I.
FIG. 5 is an XRPD pattern of crystal form B of the monohydrochloride of the compound of formula I.
FIG. 6 is a DSC profile of the crystal form B of the monohydrochloride of the compound of formula I.
FIG. 7 is a TGA profile of the crystal form B of the monohydrochloride of the compound of formula I.
FIG. 8 is a PLM image of the crystal form B of the monohydrochloride of the compound of formula I.
FIG. 9 is an XRPD pattern of crystal form C of the monohydrochloride of the compound of formula I.
FIG. 10 is a DSC profile of the crystal form C of the monohydrochloride of the compound of formula I.
FIG. 11 is a TGA profile of the crystal form C of the monohydrochloride of the compound of formula I.
FIG. 12 is a PLM image of the crystal form C of the monohydrochloride of the compound of formula I.
FIG. 13 is an XRPD pattern of crystal form D of the monohydrochloride of the compound of formula I.
FIG. 14 is a DSC profile of the crystal form D of the monohydrochloride of the compound of formula I.
FIG. 15 is a TGA profile of the crystal form D of the monohydrochloride of the compound of formula I.
FIG. 16 is a PLM image of the crystal form D of the monohydrochloride of the compound of formula I.
FIG. 17 is an XRPD pattern of crystal form E of the monohydrochloride of the compound of formula I.
FIG. 18 is a graph showing the XRPD results of the crystal form C of the monohydrochloride of the compound of formula I after storage under acceleration and high temperature conditions for 1 month.
FIG. 19 is a graph showing the XRPD results of the crystal form C of the monohydrochloride of the compound of formula I after exposure to high temperature and high humidity conditions for 1 day.
FIG. 20 is a graph showing the XRPD results of the crystal form C of the monohydrochloride of the compound of formula I after exposure to high temperature and high humidity conditions for 3 days.
FIG. 21 shows the experimental data of the cynomolgus monkey plasma concentration curves in the biological examples (ng/mL).
FIG. 22 shows the experimental data of the cynomolgus monkey serum AP activity curves in the biological examples (% relative to 0 h).
FIG. 23 shows the experimental data for *Streptococcus-*induced rheumatoid arthritis in rats in the biological examples.
FIG. 24 is a single crystal pattern of the monohydrochloride of the compound of formula I.

### DETAILED DESCRIPTION

The technical solutions of the present disclosure will be further described in detail with reference to the following specific examples. It should be understood that the following examples are merely exemplary illustration and explanation of the present disclosure and should not be construed as limiting the protection scope of the present disclosure. All techniques implemented based on the contents described above of the present disclosure are encompassed within the protection scope of the present disclosure.

Unless otherwise stated, the starting materials and reagents used in the following examples are all commercially available products or can be prepared by using known methods.

### Parameters of experimental instruments

### X-ray powder diffraction (XRPD)

The apparatus was Shimadzu XRD-6000, and samples were scanned according to the following parameters:
the ray source was Cu-Kα target (1.54056 Å);
the minimum operating voltage and current of the light tube were 40 kV and 30 mA, respectively;
the 2-Theta values of the sample scanning range were from 2° to 50°; the scanning speed was 5 deg/min.

### Thermogravimetric analysis (TGA)

About 5 mg of a sample was weighed out and added to a crucible, the system was heated from 30 °C to 300 °C under nitrogen atmosphere with a heating rate of 20 °C/min, and the temperature was kept at 300 °C for 1 min.

### Differential scanning calorimeter (DSC)

About 1-5 mg of a powder sample was weighed out and placed into a closed aluminum crucible with a pinhole on a crucible lid. Under nitrogen atmosphere, the system was heated from 30 °C to 300 °C for differential scanning calorimetry, and the temperature was kept at 300 °C for 1 min. The heating rate was 20 °C/min.

### Polarizing microscope (PLM)

A sample was dispersed in a medium (silicone oil) and observed using a 10× eyepiece lens and a 10× objective lens, and the images were recorded using a camera-computer system.

### Dynamic vapor sorption (DVS)

Under a cycle of 0%-95%-0% relative humidity (RH), about 10 mg of a sample was weighed out and subjected to a moisture sorption/desorption property test at 25 °C. The parameters are as follows:

| **Setting** | **Parameter** |
|---|---|
| Sample chamber temperature | 25 °C |
| Equilibrium condition | dm/dt: 0.01%/min |
| Humidity range, RH (%) | 0%-95%-0% RH |
| Measurement step length, RH (%) | 5% RH |
| Sample amount | 10-20 mg |

### Categories of hygroscopicity:

| **Hygroscopicity grading** | **Vapor sorption standard*** |
|---|---|
| Deliquescence | Absorbing sufficient water to form a solution |
| Very hygroscopic | W≥15% |
| Hygroscopic | 2%≤W<15% |
| Slightly hygroscopic | 0.2%≤W<2% |
| Less hygroscopic | W<0.2% |

| | |
|---|---|
| "*": under the conditions of 25±1 °C and 80±2% RH (European Pharmacopoeia 10.0) "W": the hygroscopic weight gain at 80% RH. | |

### Single crystal test apparatus and conditions

| | |
|---|---|
| Instrument model: D8 Venture | |
| Instrument parameters: | |
| light source: Mo target | X-ray: Mo-Kα (= 0.71073 Å) |
| detector: a CMOS plane detector | resolution: 0.80 Å |
| current and voltage: 50 kV, 1.4 mA | exposure time: 10 s |
| distance between the plane detector and the sample: 40 mm | test temperature: 170(2) K |

### Description of abbreviations

40 °C/75% RH refers to a condition of 40 °C and 75% humidity.
40 °C/75% RH-closed refers to closed storage under a condition of 40 °C and 75% humidity.
40 °C/75% RH-open refers to open storage under a condition of 40 °C and 75% humidity.
60 °C-closed refers to closed storage at 60 °C.
40 °C/75% RH-closed-2wks refers to closed storage under a condition of 40 °C and 75% humidity for 2 weeks.
40 °C/75% RH-open-2wks refers to open storage under a condition of 40 °C and 75% humidity for 2 weeks.
60 °C-closed-2wks refers to closed storage at 60 °C for 2 weeks.
Initial refers to an initial state.
SGF refers to simulated gastric fluid.
FaSSIF refers to simulated intestinal fluid in a fasted state .
FeSSIF refers to simulated intestinal fluid in a fed state .
1d refers to 1 day; 3d refers to 3 days.

### Preparation Example 1: Preparation of Compound of Formula I

### Reaction schemes for synthesis of compound of formula I and intermediate b

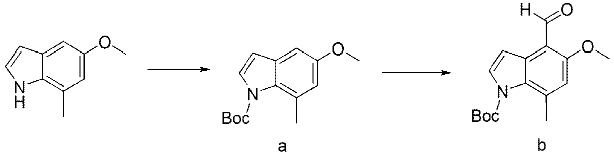

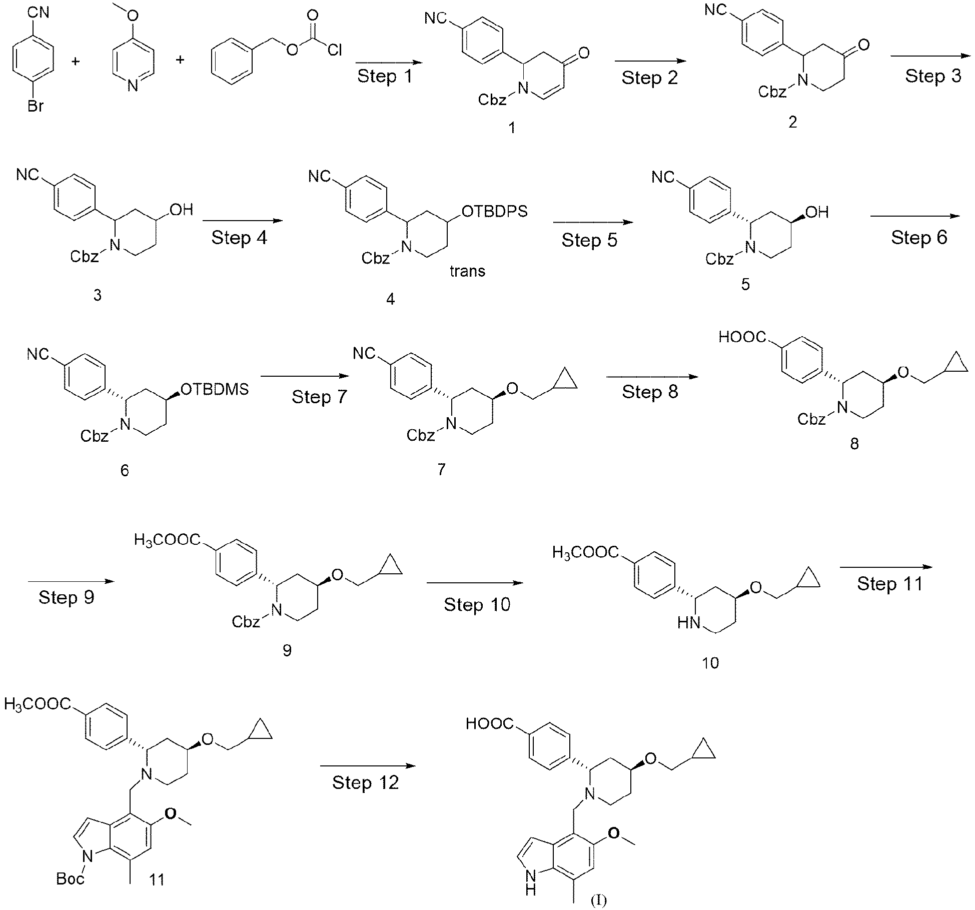

### Preparation of intermediate b

To a 250 mL single-neck flask were successively added dichloromethane (50 mL), 5-methoxy-7-methyl-1*H*-indole (3 g), Boc anhydride (5.68 g), 4-dimethylaminopyridine (227 mg), and triethylamine (2.26 g). The mixture was reacted at room temperature for 16 h. After the reaction was completed, the reaction solution was quenched by adding saturated ammonium chloride solution (5 mL) and extracted three times with dichloromethane (20 mL). The combined organic phase was washed with water (5 mL), dried over anhydrous sodium sulfate and filtered. The filtrate was concentrated. The residue was purified by column chromatography (petroleum ether:ethyl acetate = 10:1) to give intermediate a (4.6 g, yield: 94%). MS m/z (ESI): 262.0[M+1].

To a 250 mL single-neck flask was successively added dichloromethane (80 mL), N-methylformanilide (3.8 g), and oxalyl chloride (3.6 g). The reaction mixture was stirred at room temperature for 3 h. Then the reaction temperature was lowered to -14 °C, and intermediate a (2.5 g) was added. The reaction system was naturally raised to room temperature and stirred at room temperature for 1 h. After the reaction was completed, the reaction solution was poured into ice water (100 mL), and the resulting mixture was extracted three times with dichloromethane (100 mL). The combined organic phase was washed twice with water (10 mL), dried over anhydrous sodium sulfate and filtered. The filtrate was concentrated. The residue was purified by column chromatography (petroleum ether:ethyl acetate = 20:1) to give intermediate b (1.3 g, yield: 47%). MS m/z (ESI): 290.0[M+1]. ¹H NMR (400 MHz, CDCl₃) *δ* 10.65 (s, 1H), 7.65 (d, *J =* 3.4 Hz, 1H), 7.49 (d, *J =* 3.4 Hz, 1H), 6.76 (s, 1H), 3.98 (s, 3H), 2.70 (s, 3H), 1.65 (s, 9H).

### Preparation of compound of formula I

### Step 1:

To a 3 L three-necked flask was successively added tetrahydrofuran (150 mL) and 4-bromoxynil (50 g). Isopropylmagnesium chloride lithium chloride complex (1.3 M, 210 mL) was slowly added to the reaction system under nitrogen atmosphere. The mixture was reacted at room temperature for 2 h. Then anhydrous tetrahydrofuran (500 mL) was added to the reaction system for dilution, and the mixture was cooled to -5 °C. 4-Methoxypyridine (25 mL) was added, and benzyl chloroformate (35 mL) was slowly added dropwise (the system temperature was maintained at below 0 °C). After the dropwise addition was completed, the reaction mixture was stirred at 0 °C for 2 h, and then heated to room temperature and reacted at room temperature for another 16 h. After the reaction was completed, 6 M hydrochloric acid (150 mL) was added. The mixture was stirred for half an hour, and water (1000 mL) was added for dilution. The resulting mixture was extracted twice with ethyl acetate (500 mL). The combined organic phase was washed with saturated brine (50 mL), dried over anhydrous sodium sulfate and filtered. The filtrate was concentrated, and the resulting crude product was purified by column chromatography (petroleum ether:ethyl acetate = 3:1 to 1:1) to give compound 1 (23 g, yield: 23%).

MS m/z (ESI): 333.0[M+1].

### Step 2:

28 g of 46 g of compound 1 obtained in two batches in step 1 was taken out and successively added, together with zinc powder (55 g) and acetic acid (200 mL), to a 500 mL single-neck flask. The reaction mixture was heated to 100 °C and stirred at this temperature for 16 h. After the reaction was completed, the mixture was filtered. Water (500 mL) was added to the filtrate for dilution, and the resulting mixture was extracted with ethyl acetate (500 mL). The organic phase was washed twice with saturated aqueous sodium bicarbonate solution (500 mL), washed once with saturated brine (100 mL), dried over anhydrous sodium sulfate and filtered. The filtrate was concentrated under reduced pressure to give compound 2 (26 g, yield: 73%).

MS m/z (ESI): 334.8[M+1].

### Step 3:

To a 500 mL single-neck flask was successively added tetrahydrofuran (100 mL), ethanol (100 mL), and compound 2 (26 g), and sodium borohydride (2 g) was added in batches. The mixture was reacted at room temperature for 2 h. After the reaction was completed, the system was cooled to 0 °C, and saturated aqueous ammonium chloride solution (30 mL) was added until the temperature did not increase any more. Water (500 mL) was added for dilution, and the resulting mixture was extracted twice with ethyl acetate (200 mL). The combined organic phase was washed with saturated brine (500 mL), dried over anhydrous sodium sulfate and filtered. The filtrate was concentrated under reduced pressure to give compound 3 (25 g, yield: 76%).

MS m/z (ESI): 336.9[M+1].

### Step 4:

Dichloromethane (200 mL) was added to a 500 mL single-neck flask, and then compound 3 (25 g), imidazole (6.6 g), and *tert*-butyldiphenylchlorosilane (25 g) were successively added. The mixture was reacted at room temperature for 2 h. After the reaction was completed, the reaction solution was washed with water (500 mL), dried over anhydrous sodium sulfate and filtered. The filtrate was concentrated under reduced pressure. The residue was purified by column chromatography (petroleum ether: ethyl acetate = 10:1) to give compound 4 (5.7 g, yield: 13%, Rf = 0.55; trans-isomer Rf = 0.50).

MS m/z (ESI): 597.0[M+23].

### Step 5:

To a 250 mL single-neck flask was successively added compound 4 (5 g) and a solution of tetrabutylammonium fluoride in tetrahydrofuran (1 M, 30 mL). The mixture was reacted at room temperature for 2 h. After the reaction was completed, water (100 mL) was added for dilution, and the resulting mixture was extracted three times with ethyl acetate (50 mL). The combined organic phase was washed with saturated brine (100 mL), dried over anhydrous sodium sulfate and filtered. The filtrate was concentrated under reduced pressure. The residue was purified by column chromatography (petroleum ether:ethyl acetate = 3:1 to 0:1) to give a racemate. The racemate was subjected to SFC chiral resolution (Apparatus: SFC Thar prep 80; Column: CHIRALPAK AD-H, 250 mm × 20 mm, 5 µm; Modifier: 35% methanol (0.2% aqueous ammonia); column temperature: 40 °C; column pressure: 60 bar; wavelength: 214/254 nm; flow rate: 40 g/min; Rt = 4.78 min) to give compound 5 (1.2 g, yield: 41%).

MS m/z (ESI): 358.8[M+23].

### Step 6:

To a solution of compound 5 (1200 mg) in N,N-dimethylformamide (10 mL) was added imidazole (486 mg) and *tert*-butyldimethylchlorosilane (593 mg). The reaction mixture was stirred at room temperature for 2 h. After the reaction was completed, water (100 mL) was added to the reaction mixture for dilution, and the resulting mixture was extracted with ethyl acetate (50 mL). The organic phase was washed once with saturated brine (50 mL), dried over anhydrous sodium sulfate and filtered. The filtrate was directly concentrated to give compound 6 (600 mg, yield: 90%).

MS m/z (ESI): 472.8[M+23].

### Step 7:

700 mg of compound 6 was taken out of 1.2 g of compound 6 obtained in two batches in step 6 and added to dichloromethane (10 mL) at room temperature. Under nitrogen atmosphere and at -78 °C, cyclopropanecarboxaldehyde (110 mg) and trimethylsilyl trifluoromethanesulfonate (35 mg) were added to the reaction solution. The reaction system was maintained at -78 °C and stirred for one hour. Then triethylsilane (180 mg) was added. The reaction mixture was naturally heated to room temperature and stirred at this temperature for another 16 h. After the reaction was completed, the reaction solution was quenched by adding saturated aqueous sodium bicarbonate solution (20 mL). Water (10 mL) was added for dilution, and the resulting mixture was extracted with dichloromethane (10 mL). The organic phase was washed once with water (10 mL), dried over anhydrous sodium sulfate and filtered. The filtrate was concentrated. The residue was purified by column chromatography (petroleum ether:ethyl acetate = 3:1) to give compound 7 (400 mg, yield: 46%).

MS m/z (ESI): 390.9[M+1].

### Step 8:

To a 50 mL single-neck flask was successively added compound 7 (400 mg), isopropanol (2 mL), water (3 mL), and sodium hydroxide (400 mg). The reaction mixture was heated to 100 °C and stirred at this temperature for 16 h. After the reaction was completed, diluted hydrochloric acid (1 M) was added to the reaction solution in an ice bath to adjust the pH to 5-6. Water (5 mL) was added for dilution, and the resulting mixture was extracted with ethyl acetate (5 mL). The organic phase was washed once with saturated brine (5 mL), dried over anhydrous sodium sulfate and filtered. The filtrate was concentrated at 45 °C to give compound 8 (200 mg, yield: 33%).

MS m/z (ESI): 431.8[M+23].

### Step 9:

Potassium carbonate (135 mg) and iodomethane (140 mg) were added to a solution of compound 8 (200 mg) in acetonitrile (5 mL). The reaction solution was heated to 50 °C and stirred at this temperature for 16 h. After the reaction was completed, the reaction solution was directly concentrated. The residue was purified by column chromatography (petroleum ether:ethyl acetate = 3:1) to give compound 9 (180 mg, yield: 40%).

MS m/z (ESI): 445.8[M+23].

### Step 10:

To a solution of compound 9 (180 mg) in tetrahydrofuran (3 mL) was added palladium/carbon (50 mg). The reaction solution was subjected to a catalytic hydrogenation reaction under hydrogen atmosphere at room temperature for 2 h. After the reaction was completed, the reaction solution was filtered. The filtrate was directly concentrated to give compound 10 (120 mg, yield: 54%).

MS m/z (ESI): 290.0[M+1].

### Step 11:

Compound 10 (120 mg) was added to a solution of intermediate b (119 mg) in 1,2-dichloroethane (5 mL). The reaction mixture was stirred at room temperature for 8 h. Then sodium borohydride acetate (261 mg) was added, and the mixture was stirred at room temperature for another 16 h. After the reaction was completed, the reaction solution was directly concentrated. The residue was purified by column chromatography (dichloromethane:methanol = 20:1) to give compound 11 (200 mg, yield: 26%).

MS m/z (ESI): 562.8[M+1].

### Step 12

To a 50 mL single-neck flask were successively added methanol (2 mL), water (2 mL), compound 11 (200 mg), and sodium hydroxide (150 mg). The reaction mixture was heated to 75 °C and stirred at this temperature for 3 h. After the reaction was completed, diluted hydrochloric acid (1 M) was added to the reaction solution in an ice bath to adjust the pH to 7. Then the mixture was directly concentrated under reduced pressure and purified by Prep-HPLC (column: Gemini-C18 150 × 21.2 mm, 5 µm; mobile phase: acetonitrile-water (0.1% formic acid); gradient: 20%-40%) to give the compound of formula I (30.6 mg, yield: 18%; containing 0.5 equivalents of formic acid).

MS m/z (ESI): 448.9[M+1].

¹H NMR (400 MHz, CD₃OD): δ8.18 (d, *J =* 7.7 Hz, 2H), 7.69 (d, *J =* 7.7 Hz, 2H), 7.32 (s, 1H), 6.76 (s, 1H), 6.34 (s, 1H), 4.88-4.61 (m, 1H), 4.44-4.07 (m, 2H), 3.95-3.81 (m, 1H), 3.75 (s, 3H), 3.63-3.47 (m, 1H), 3.46-3.33 (m, 3H), 2.50 (s, 3H), 2.35-2.14 (m, 2H), 2.13-1.94 (m, 2H), 1.23-1.04 (m, 1H), 0.58 (d, *J =* 7.2 Hz, 2H), 0.28 (d, *J =* 3.8 Hz, 2H).

Unless otherwise stated, all compounds of formula I mentioned below are the compound of formula I prepared by the method described above or by repeating the method described above.

### Example 1. Preparation Method for Monohydrochloride of Compound of Formula I

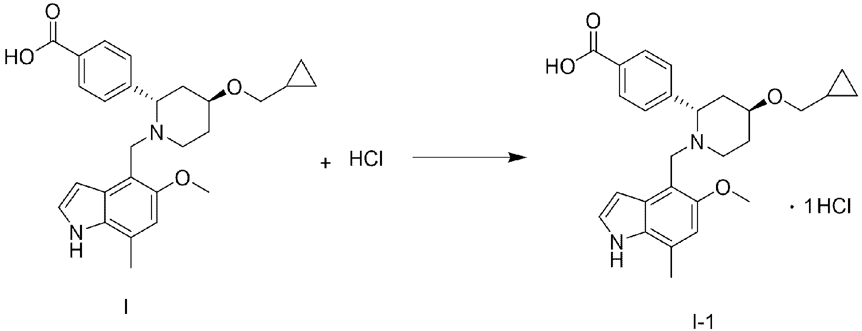

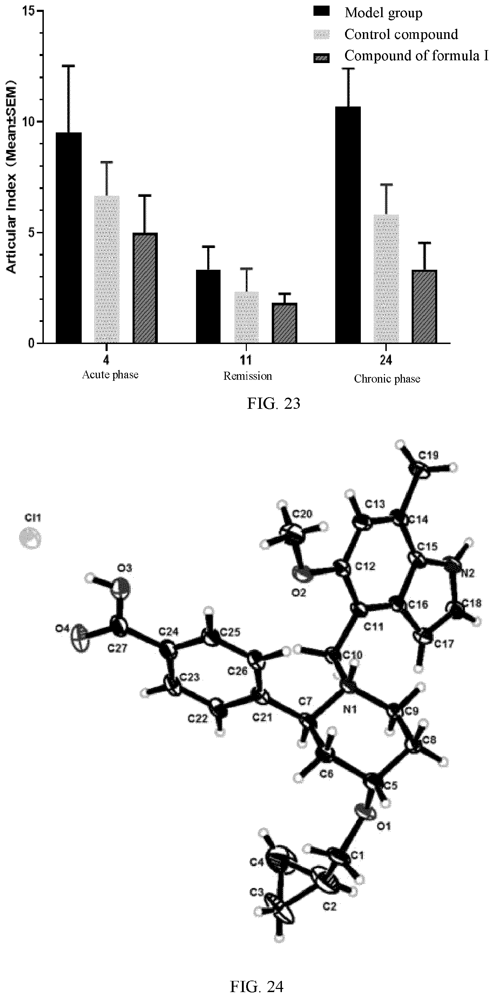

After the compound of formula I was prepared in multiple batches, 400 mg of the compound of formula I was taken out, and isopropanol (8 mL) was added. The mixture was heated for dissolution at 50 °C. A solution of hydrogen chloride in isopropanol (460 µL, 2 mol/L) was slowly added dropwise, and the mixture was stirred for half an hour. *n*-Heptane (8 mL) was then added, and the mixture was stirred for another 2 h and filtered. The filter cake was dried in vacuum under reduced pressure at 50 °C to give monohydrochloride I-1 of the compound of formula I (390 mg, yield: 90%). 50 mg of monohydrochloride I-1 of the compound of formula I was taken out and added to a 4 mL sample bottle, and methanol (0.5 mL) was added to dissolve the compound. The sample bottle was open and placed in a 40 mL sample bottle containing ethyl acetate (5 mL), and the 40 mL sample bottle was kept closed. The sample bottles were left to stand, and the two solvents were diffused slowly to give a single crystal. The single crystal structure is shown in FIG. 24. The single crystal was tested. The single crystal data for monohydrochloride I-1 of the compound of formula I are as follows:

| | |
|---|---|
| Cl·C₂₇H₃₃N₂O₄ | *D*ₓ = 1.273 Mg m⁻³ |
| *Mᵣ* = 485.00 | Mo *K*α radiation, λ = 0.71073 Å |
| Orthorhombic, space group *P*2₁2₁2₁ | Unit cell parameter, 3954 reflection points |
| *a* = 9.4704 (18) Å | θ= 2.5-23.7° |
| *b* = 15.324 (4) Å | µ = 0.19 mm⁻¹ |
| *c* = 17.437 (4) Å | *T =* 170 K |
| *V =* 2530.5 (10) Å³ | Massive, colorless |
| *Z* = 4 | 0.15 × 0.08 × 0.05 mm |
| *F*(000) = 1032 | |

### Example 2. Preparation Method for Phosphate of Compound of Formula I

The compound of formula I was prepared in multiple batches, and 440 mg of the compound of formula I was taken out. Acetone (5 mL) was added, and the mixture was heated to 40 °C and ultrasonically dissolved. A solution of phosphoric acid in methanol (460 µL, 2 mol/L) was then slowly added dropwise. A viscous solid was found, and then acetone (5 mL) was added. The mixture was stirred at room temperature for 4 h, filtered and washed. The filter cake was dried in vacuum under reduced pressure at 50 °C to give phosphate of the compound of formula I (462 mg, yield: 87%).

### Example 3. Preparation Method for Maleate of Compound of Formula I

The compound of formula I was prepared in multiple batches, and 400 mg of the compound of formula I was taken out. Ethyl acetate (15 mL) was added, and the mixture was heated to 50 °C to dissolve the compound. Maleic acid powder (109 mg) was then added, and the mixture was stirred at room temperature for 2-3 h and filtered. The filter cake was dried in vacuum under reduced pressure at 50 °C to give maleate of the compound of formula I (470 mg, yield: 91%).

### Example 4. Stability Tests of Monohydrochloride, Phosphate, and Maleate of Compound of Formula I

The stability of the compound of formula I, the monohydrochloride of the compound of formula I in Example 1, the phosphate of the compound of formula I in Example 2, and the maleate of the compound of formula I in Example 3 were investigated.

Stability investigation conditions: 40 °C/75% RH-closed, 40 °C/75% RH-open, and 60 °C-closed; stability investigation content: changes in related substances and crystal forms.

Detection of related substances: about 6-7 mg of a sample was separately weighed out and added to a 10 mL volumetric flask; 50% aqueous acetonitrile solution was added for dissolution; the mixture was diluted to volume; and 10 µL of the sample was injected. The chromatographic conditions are shown in Table 6.

The experimental results of the stability investigation of the compound of formula I and the hydrochloride, phosphate and maleate thereof are shown in Table 7.

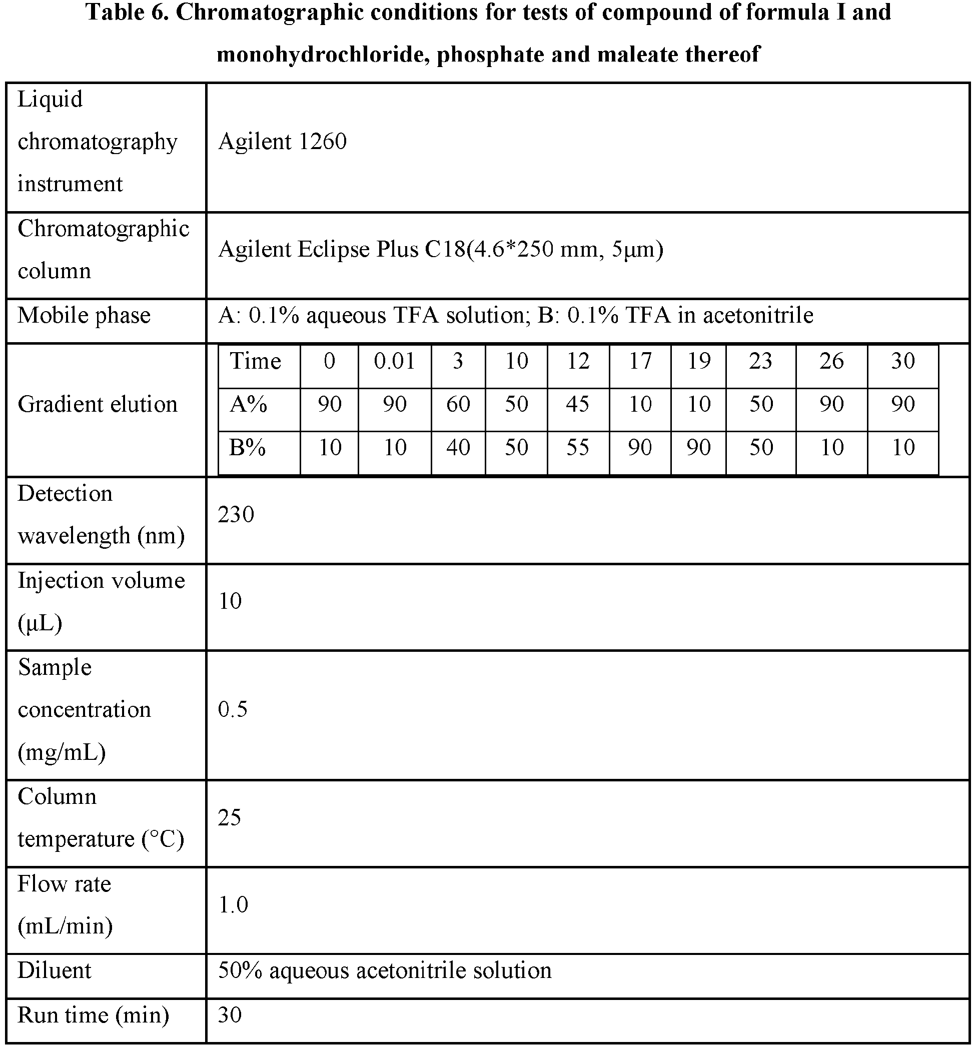

**Table 7. Experimental results of stability investigation of compound of formula I and monohydrochloride, phosphate and maleate thereof**

| **Salt form** | **Stability condition** | **Appearance** | **Purity%** | **Total impurity%** | **Maximum single impurity%** |
|---|---|---|---|---|---|
| Compound of formula I | Initial | Off-white powder | 99.28 | 0.72 | 0.10 |
| | 40°C/75%RH-closed-2wks | Off-white powder | 99.23 | 0.77 | 0.16 |
| | 40°C/75%RH-open-2wks | Off-white powder | 99.24 | 0.76 | 0.17 |
| | 60°C-closed-2wks | Off-white powder | 98.95 | 1.05 | 0.33 |
| Compound of formula I Monohydrochloride | Initial | White powder | 99.59 | 0.41 | 0.14 |
| | 40°C/75%RH-closed-2wks | White powder | 99.57 | 0.43 | 0.11 |
| | 40°C/75%RH-open-2wks | White powder | 99.59 | 0.41 | 0.10 |
| | 60°C-closed-2wks | White powder | 99.59 | 0.41 | 0.13 |
| Compound of formula I Phosphate | Initial | White powder | 99.75 | 0.25 | 0.07 |
| | 40°C/75%RH-closed-2wks | White powder | 99.63 | 0.37 | 0.09 |
| | 40°C/75%RH-open-2wks | White powder | 99.64 | 0.36 | 0.09 |
| | 60°C-closed-2wks | White powder | 99.62 | 0.38 | 0.08 |
| Compound of formula I Maleate | Initial | White powder | 99.80 | 0.20 | 0.08 |
| | 40°C/75%RH-closed-2wks | White powder | 99.78 | 0.22 | 0.08 |
| | 40°C/75%RH-open-2wks | Yellow and clumped | 99.64 | 0.36 | 0.09 |
| | 60°C-closed-2wks | White powder | 99.69 | 0.31 | 0.09 |

As can be seen from the results in Table 7, the compound of formula I and the monohydrochloride, phosphate and maleate thereof were all stable, and particularly, the monohydrochloride of the compound of formula I was more stable.

### Example 5. Solubility Tests of Compound of Formula I and Hydrochloride and Phosphate Thereof

The solubility of the compound of formula I, the monohydrochloride of the compound of formula I, and the phosphate of the compound of formula I in water, SGF, FaSSIF, and FeSSIF was investigated at 37 °C.

Experimental procedures: 30 mg (in water) or 15 mg of a sample was weighed out and added to a 4 mL vial; 3 mL of a medium to be tested (water, SGF, FaSSIF, or FeSSIF) was added; the mixture was continuously stirred at 37 °C; 0.5 mL of the sample was separately collected at 1 h and 24 h; centrifugation was performed at 12000 rpm for 10 min; the supernatant was diluted with 50% aqueous acetonitrile solution to an appropriate multiple; and the concentration of the supernatant was determined. The chromatographic conditions for the solubility tests are shown in Table 8.

Control and linearity: 10 mg of the compound of formula I was weighed out and added to a 50 mL volumetric flask, 50% aqueous acetonitrile solution was added for dissolution, and the mixture was diluted to volume. Two parts were prepared in parallel. The control of the compound of formula I was taken and diluted with 50% aqueous acetonitrile solution to 100 µg/mL, 50 µg/mL, and 10 µg/mL, 5 µL of the sample was injected, and a standard curve was plotted.

The solubility test results of the compound of formula I and the hydrochloride and phosphate thereof are shown in Table 9.

**Table 8. Chromatographic conditions for solubility test of compound of formula I**

| | |
|---|---|
| Liquid chromatography instrument | Agilent 1260 |
| Chromatographic column | Waters Sunfire C18 (4.6*150 mm, 5µm) |
| Mobile phase | A: 0.1% aqueous TFA solution; B: 0.1% TFA solution in acetonitrile |
| Isocratic elution | A:B=60:40 |
| Detection wavelength (nm) | 230 |
| Injection volume (µL) | 5 |
| Sample concentration (mg/mL) | 0.5 |
| Column temperature (°C) | 40 |
| Flow rate (mL/min) | 1.0 |
| Diluent | 50% aqueous acetonitrile solution |
| Run time (min) | 30 |

**Table 9. Solubility test results of compound of formula I and hydrochloride and phosphate thereof**

| **Sample** | **Medium** | **Sample amount (mg)** | **Medium volume (mL)** | **Solubility (mg/mL) (1 h)** | **Solubility (mg/mL) (24 h)** | **Sample appearance** | **Final pH** |
|---|---|---|---|---|---|---|---|
| Compound of formula I | Water | 31.89 | 3 | 0.37 | 0.31 | White suspension | 8.73 |
| | SGF | 15.73 | 3 | 5.51 | 5.48 | Pale purple solution | 1.74 |
| | FaSSIF | 16.35 | 3 | 0.22 | 0.19 | White suspension | 6.44 |
| | FeSSIF | 16.45 | 3 | 0.37 | 0.35 | White suspension | 5.03 |
| Compound of formula I Hydrochloride | Water | 33.71 | 3 | 11.74 | 11.14 | Light grey suspension | 2.49 |
| | SGF | 16.29 | 3 | 5.90 | 5.97 | Pale purple solution | 1.52 |
| | FaSSIF | 16.73 | 3 | 0.64 | 0.39 | White suspension | 3.83 |
| | FeSSIF | 15.82 | 3 | 0.74 | 0.44 | White suspension | 4.85 |
| Compound of formula I Phosphate | Water | 34.27 | 3 | 8.07 | 6.24 | Light grey suspension | 2.53 |
| | SGF | 17.56 | 3 | 5.35 | 5.55 | Pale purple solution | 1.61 |
| | FaSSIF | 17.33 | 3 | 0.45 | 0.27 | White suspension | 4.76 |
| | FeSSIF | 17.69 | 3 | 0.64 | 0.40 | White suspension | 4.84 |

As can be seen from Table 9, after the compound of formula I was salified, the solubility in water was increased.

### Control compound

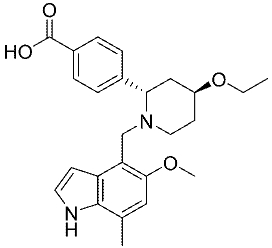

To a 50 mL single-neck flask were added methanol (3 mL), water (1 mL), intermediate 1 (160 mg), and sodium hydroxide (230 mg). The mixture was reacted at room temperature for 16 h. After the reaction was completed, water (10 mL) was added for dilution, and the pH was adjusted to 7-8 with a diluted hydrochloric acid solution (1 M). The solvent was removed under reduced pressure (water bath: 45 °C). The residue was purified by preparative high-pressure liquid chromatography (column: Gemini-C18, 150 × 21.2 mm, 5 µm; mobile phase: acetonitrile-water (0.1% formic acid); gradient: 15%-30%) to give the control compound (29 mg, yield: 24%). MS m/z (ESI): 423.1 [M+1]. ¹H NMR (400 MHz, DMSO-*d₆*) δ 8.17 (d, J = 8.4 Hz, 2H), 7.67 (d, J = 8.4 Hz, 2H), 7.33 (t, J = 2.8 Hz, 1H), 6.78 (s, 1H), 6.35 (s, 1H), 4.82 - 4.67 (m, 1H), 4.40 - 4.17 (m, 2H), 3.90 - 3.81 (m, 1H), 3.77 (s, 3H), 3.62 (q, J = 6.8 Hz, 2H), 3.57 - 3.50 (m, 1H), 3.45 - 3.35 (m, 1H), 2.52 (s, 3H), 2.32 - 2.22 (m, 2H), 2.14 - 1.96 (m, 2H), 1.32 (t, J = 6.8 Hz, 3H).

### Biological Example 1

### 1. Optical surface plasmon resonance (SPR) binding capacity assay

An SPR experiment was carried out at 25 °C. In the experiment, a PBS buffer supplemented with 0.05% (v/v) P20 and 5% DMSO was used as a running buffer, and the analytical instrument Biacore 8K of GE Healthcare was used. A CM7 chip (GE Healthcare) was activated with 400 mM EDC and 100 mM NHS at a flow rate of 30 µL/min for 420 s. Complement factor B was diluted to 50 µg/mL with 10 mM sodium acetate (pH 4.0) and then covalently immobilized to the assay chip by coupling at a flow rate of 10 µL/min for 1200 s (protein immobilization level at 25000 RU). Then the assay chip was treated with 1 M ethanolamine hydrochloride at a flow rate of 10 µL/min for 300 s for chip blocking. The concentration of the test compound was 500 µM, the binding time was 120 s, and the dissociation time was 300 s. Data analysis was performed using a 1:1 binding model (Biacore Insight Evalution Software, Version2.0.15.12933).

### Results:

The experimental results are shown in Table 10. At a concentration of 500 µM, the compound of formula I had more significant binding capacity to the target protein and was significantly superior to that of the control compound, which indicates that the compound of formula I has relatively good binding capacity to the target protein.

**Table 10**

| Sample | Rmax (RU) |
|---|---|
| Compound of formula I | 274.2 |
| Control compound | 33.7 |

### 2. TR-FRET binding capacity assay

Competitive binding experiments using a small-molecule inhibitor fluorescently labeled with Cy5 as the probe were carried out to screen compounds for inhibitory activity against human complement factor B. After complement factor B and EZ-Link^{™} Sulfo-NHS-LC-LC-Biotin in a ratio of 1:2 were incubated on ice for 1 h, 1 M Tris (pH 7.5) was added to terminate the reaction. The mixture was then purified twice through a 2 mL Zeba^{™} desalt spin column to give a biotin-labeled complement factor B (EZ-LinkTM Sulfo-NHS-LC-Biotin instructions). In the experiments, biotin-labeled complement factor B at a final concentration of 10 nM was pre-incubated with different concentrations of compounds in the buffer at room temperature for 1 h. The reaction was initiated by adding a Cy5 fluorescently labeled probe and europium chelate-labeled streptavidin (petroleum ether rkin Elmer, #AD0060) at final concentrations of 75 nM and 5 nM, respectively. Kinetic readings were taken on a microplate reader (excitation light at 337 nm, emitted light at 665 nm, 70 µs time-gated) and time-resolved fluorescence resonance energy transfer (TR-FRET) data were read to determine IC₅₀.

### 3. Complement system hydrolysis C3 activity assay

Test compounds were 3-fold diluted from a starting concentration of 10 µM to 7 concentrations, and single-well assays were performed. Test compounds were diluted in a 96-well plate with DMSO into solutions with 1000× final concentration and then diluted with Diluent (WIESLAB ^{®} COMPLEMENT SYSTEM ALTERNATIVE PATHWAY AP330) into solutions with 5× final concentration. 30 µL was transferred into a 96-well plate, and 120 µL of ready-to-use serum was added. The plate was incubated at room temperature for 15 min. To a positive control well was added 30 µL of 5‰ DMSO and 120 µL of ready-to-use serum. To a negative control well was added 30 µL of 5‰ DMSO and 120 µL of Diluent. (3) 100 µL of the mixture was added to the reaction plate, and the plate was incubated at 37 °C for 60 min. The liquids in the wells were discarded, and each well was washed 3 times with 300 µL of washing liquid. To each well was added 100 µL of Conjugate (WIESLAB ^{®} COMPLEMENT SYSTEM ALTERNATIVE PATHWAY AP330). The plate was incubated at room temperature for 30 min. The liquids in the wells were discarded, and each well was washed 3 times with 300 µL of washing liquid. Then 100 µL of substrate was added to each well. The plate was incubated at room temperature for 30 min. OD405 values were read using a microplate reader (Perkin Elmer, EnSight).

### 4. Complement hemolytic activity assay

The hemolysis experiment was carried out by referring to the description in the document Xuan Yuan et al., Haematologica. (2017) 102:466-475. Prior to the experiment, the optimal concentration of normal human serum (NHS) required to achieve 100% lysis of rabbit erythrocytes (REs) was obtained by titration testing. In this experiment, NHS was diluted in a GVB0 buffer (0.1% gelatin, 5 mM Veronal, 145 mM NaCl, 0.025% NaN₃, pH 7.3, Complement technology) containing 10 mM Mg-EGTA and incubated with various concentration gradients of test compounds at 37 °C for 15 min. REs (collected from healthy Japanese big-ear white rabbits) freshly suspended in a GVB0 buffer containing 10 mM Mg-EGTA were added to a final concentration of 1 × 10⁸ cells/mL and incubated at 37 °C for 30 min. A GVB0 buffer containing 10 mM Mg-EGTA and containing NHS and RE but no test compound was used as a positive control group (100% lysis). A GVB0 buffer containing 10 mM Mg-EGTA and containing inactivated NHS (heated at 56 °C for 30 min or at 65 °C for 5 min) and RE but no test compound was used as a negative control group (0% lysis). The sample was centrifuged at 2000 g for 5 min, and the supernatant was collected. Absorbance at 415 nm (A415) was measured using a microplate reader (Molecular Devices, SpectraMax i3X). IC₅₀ values were calculated from percent hemolysis as a function of test compound concentration by non-linear regression.

### Results:

The experimental results are shown in Table 11. The compound of formula I had significantly better inhibitory activity against complement factor B in human serum than that of the control compound, which indicates that the compound of the present disclosure can relatively well inhibit the activity of complement factor B in human serum and prevent hemolysis caused by its attack on rabbit erythrocytes.

**Table 11**

| Sample | Hemolysis IC₅₀ (nM) |
|---|---|
| Compound of formula I | 87.9 |
| Control compound | 379.4 |

### 5. Liver microsomal stability experiment

### (1) Buffer preparation

A 0.1 M solution of dipotassium phosphate in distilled water (containing 1 mM ethylenediaminetetraacetic acid) was taken. Then the pH was adjusted to 7.4 with a 0.1 M solution of potassium dihydrogen phosphate in distilled water (containing 1 mM ethylenediaminetetraacetic acid).

### (2) Microsome sources and preparation of working solution

### Microsome sources:

Rat: SD Rat Liver Microsomes, Cat. No.: LM-DS-02M, RILD Research Institute for Liver Diseases (Shanghai) Co. Ltd.
Monkey: Cynomolgus Monkey Liver Microsomes, Cat. No.: LM-SXH-02M, RILD Research Institute for Liver Diseases (Shanghai) Co. Ltd.
Human: Pooled Human Liver Microsomes (Mongolian), Cat. No.: LM-R-02M, RILD Research Institute for Liver Diseases (Shanghai) Co. Ltd.

### Preparation of working solution

A 10 mM solution of each of the control compound and test compounds was prepared in DMSO. Then 10 µL of the solution was added to 190 µL of acetonitrile to form a 0.5 mM stock solution. 1.5 µL of the 0.5 mM stock solution was measured out, and 18.75 µM of 20 mg/mL liver microsomes and 479.75 µL of the buffer were added. (The actual preparation amount can be adjusted according to use).

### (3) Procedures

10 mg/mL of reduced coenzyme II (NADPH) was prepared in the buffer. A 96-well plate was placed on ice. Wells corresponding to different time points (0, 10, 30, 60 and 90 min, Non-NADPH) were set for each compound. 30 µL of the working solution was added to each well. For 0 min wells, 155 µL of glacial acetonitrile solution (the internal standard concentration was 1 µM) was added first, and after the mixtures were well mixed using a pipette, 15 µL of NADPH (10 mg/mL) was added. Before the reactions were started, the 96-well plate was pre-incubated on a thermostatic microplate shaker at 37 °C for 5 min. Then 15 µL of NADPH (10 mg/mL) was added to each well to start the metabolic reactions. After the reactions were carried out for 10, 30, 60 and 90 min, 155 µL of glacial acetonitrile solution (the internal standard concentration was 1 µM) was added to the corresponding wells to quench the reactions. After 90 min, the reaction in the Non-NADPH system was quenched by adding 155 µL of glacial acetonitrile solution (the internal standard concentration was 1 µM). After the reaction was completed, the 96-well plate was shaken on the microplate shaker (600 rpm) for 10 min and then centrifuged at 4 °C at 4000 g for 15 min. 50 µL of the supernatant was added to another 2 mL 96-well plate, and 300 µL of deionized water was added. The mixture was analyzed on an AB SCIEX ExionLC-Triple Quad 5500 high-performance liquid chromatograph-mass spectrometer. The Analyst 1.6.3 software was used. The assay results are shown in Table 12.

**Table 12**

| Sample | MMS (rat) | | MMS (monkey) | | MMS (human) | |
|---|---|---|---|---|---|---|
| | T_{1/2} (min) | Remaining (T=90min) | T_{1/2} (min) | Remaining (T=90min) | T_{1/2} (min) | Remaining (T=90min) |
| Compound of formula I | 547.65 | 89.14% | 770.376 | 93.32% | 672.513 | 91.04% |

Experimental results: the data show that the compound of formula I has significant liver microsomal stability.

### 6. PK experiment of single intragastric administration to rats

### Experimental procedures:

Six- to nine-week-old male Wistar han rats (Shanghai Sippe-Bk Lab Animal Co., Ltd.) were fasted overnight. Each group consisted of 3 rats, and they were given the control compound and the compound of formula I, respectively, by intragastric administration at 3 mg/kg at a volume of 10 mL/kg. 0.2 mL of blood was collected from the jugular vein at each time point, anticoagulated with EDTA-K2 and immediately centrifuged at 4000 rpm at 4 °C for 5 min. The supernatant was collected. The samples were cryopreserved in a freezer at -80 °C before analysis. Time points of blood collection: before administration, 5 min, 15 min, 30 min, 1 h, 2 h, 4 h, 7 h and 24 h. The states of the animals were observed at all times after the administration. After blood collection at all of the time points was completed, the animals were euthanized. The plasma samples were analyzed by LC-MS/MS. The data were used to calculate kinetic parameters (Tmax, Cmax, T1/2 and AUC) in the WinNonlin software.

### Results:

The assay results are shown in Table 13.

**Table 13**

| Sample | | Tmax (hr) | Cmax (ng/mL) | T1/2 (hr) | AUC0-t (hr*ng/mL) | AUCinf (hr*ng/mL) |
|---|---|---|---|---|---|---|
| Compound of formula I | Mean | 0.25 | 1923.36 | 1.58 | 3120.14 | 3282.35 |
| | S.D. | 0.00 | 602.66 | 0.21 | 721.79 | 692.86 |
| Control compound | Mean | 0.33 | 783.97 | 2.42 | 2533.81 | 2726.42 |
| | S.D. | 0.14 | 166.87 | 0.69 | 260.00 | 104.81 |

### 7. PK/PD experiment of single intragastric administration to cynomolgus monkeys

### Experimental procedures:

Cynomolgus monkeys were used. Each group consisted of 3 cynomolgus monkeys, and they were given the control compound at 3 mpk, the control compound at 30 mpk, the compound of formula I at 3 mpk, and the compound of formula I at 30 mpk by intragastric administration. Blood was collected at different time points and used for drug concentration analysis and complement activity detection. The compound concentration in plasma was determined by LC-MS/MS. The complement activity in serum was determined using a wieslab assay (Svar Life Science AB, COMPL AP330 RUO) kit, Normal Human Serum (Complement Technology, NHS).

### Results:

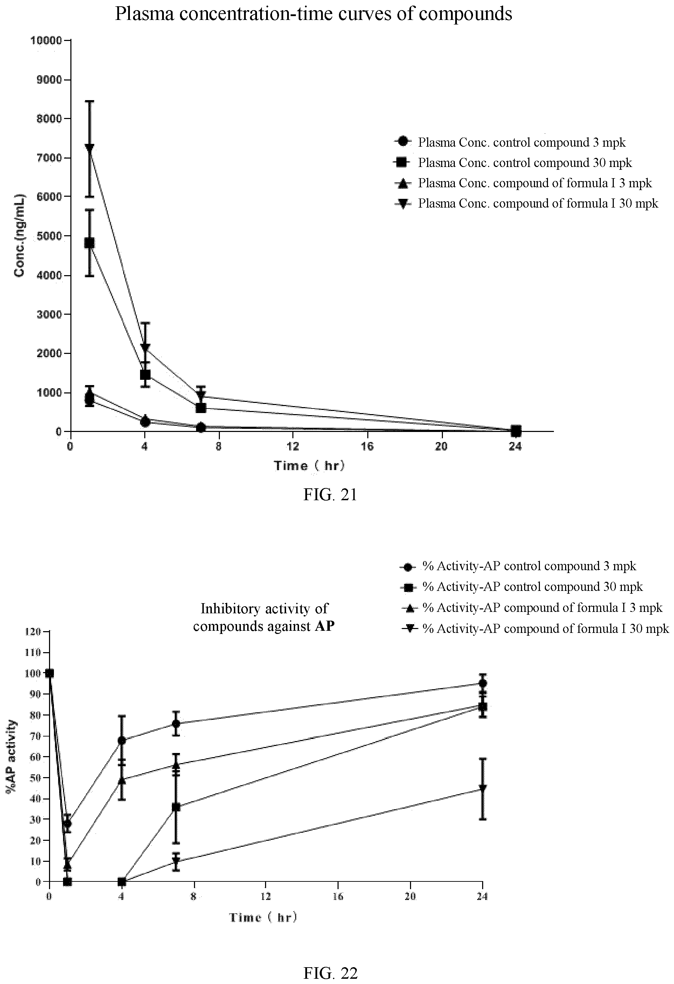

Within the ranges of concentration and time of the assay, the compound of formula I has a significantly higher mean plasma concentration than that of the control compound when administered at the same dose. The plasma concentration curves for cynomolgus monkeys are shown in FIG. 21. The inhibition of cynomolgus monkey serum AP activity is shown in FIG. 22. FIG. 22 shows that the compound of formula I can effectively inhibit the cynomolgus monkey serum AP activity.

### 8. Streptococcus-induced rheumatoid arthritis (RA) model in rats

### Experimental procedures:

In the experiment, 6- to 9-week-old female Lewis rats (Beijing Vital River) were used. Each group consisted of 6 rats, and they were given the cell wall peptidoglycan complexes of *Streptococcus* and several other species of bacteria by intraperitoneal administration (2-3 mg per rat) on D1, and were given the control compound (15 mpk) and the compound of formula I (15 mpk) by intragastric administration daily for 25 consecutive days. The arthritis in rats was scored in different phases. The scoring criteria are as follows: Scoring according to the degree of lesion (redness and swelling) was performed on a scale of 0-4 points, with a maximum score of 4 for each limb and a total maximum score of 16 for each animal's limbs. The scoring criteria are as follows: 0 points, no redness and swelling; 1 point, 1-2 red and swollen interphalangeal joints; 2 points, 3-4 red and swollen interphalangeal joints; 3 points, more than 4 red and swollen interphalangeal joints; 4 points, severe redness and swelling in toes or fingers to ankle joints or wrist joints.

**Results:**

The experimental results are shown in FIG. 23. The data show that the compound of formula I can improve the arthritis score, and that the compound of formula I has a significantly better effect than that of the control compound, which demonstrates that the compound of formula I can more effectively ameliorate *Streptococcus-*induced rheumatoid arthritis in rats.

### Example 6. Preparation Method for Crystal Form A of Monohydrochloride of Compound of Formula I

400 mg of the compound of formula I was taken out, and isopropanol (8 mL) was added. The mixture was heated for dissolution at 50 °C. A solution of hydrogen chloride in isopropanol (460 µL, 2 mol/L) was slowly added dropwise, and the mixture was stirred for half an hour. *n*-Heptane (8 mL) was then added, and the mixture was stirred for another 2 h and filtered. The filter cake was dried in vacuum under reduced pressure at 50 °C to give crystal form A of the monohydrochloride of the compound of formula I (390 mg, yield: 90%).

The crystal form A was characterized by XRPD, DSC, TGA, and PLM.

The crystal form A is an anhydrate. The locations and intensities of XRPD characteristic peaks are shown in Table 1, and the XRPD pattern is shown in FIG. 1.

DSC shows a first endothermic peak at a peak temperature raised to about 192.73 °C and a first exothermic peak at a peak temperature of about 201.78 °C, as shown in FIG. 2.

TGA shows a weight loss of about 1.41% from 90 °C to 180 °C, as shown in FIG. 3.

The PLM image shows that the sample is a crystal with irregular morphology and a size below 20 µm, as shown in FIG. 4.

In the XRPD pattern of the crystal form A expressed in terms of 2θ angles, the 2θ angles and relative intensities of the diffraction peaks are shown in Table A, wherein the 2θ angles have an error range of ±0.20°:

**Table A. XRPD analysis data for crystal form A**

| Peak number | 2θ [°] | Relative intensity (%) | Peak number | 2θ [°] | Relative intensity (%) |
|---|---|---|---|---|---|
| 1 | 8.02 | 19.6 | 16 | 26.78 | 11.9 |
| 2 | 9.66 | 100 | 17 | 27.66 | 25.6 |
| 3 | 11.62 | 38.2 | 18 | 28.44 | 7.2 |
| 4 | 12.78 | 13.9 | 19 | 28.70 | 7.7 |
| 5 | 14.32 | 13.1 | 20 | 29.04 | 9.5 |
| 6 | 16.08 | 84.5 | 21 | 29.70 | 13.9 |
| 7 | 16.84 | 24 | 22 | 30.14 | 14.6 |
| 8 | 18.10 | 64.7 | 23 | 32.38 | 10.9 |
| 9 | 19.64 | 32 | 24 | 33.82 | 6.4 |
| 10 | 21.30 | 53.5 | 25 | 35.41 | 7.3 |
| 11 | 21.68 | 42.8 | 26 | 36.89 | 5.4 |
| 12 | 23.46 | 93.7 | 27 | 37.92 | 9.3 |
| 13 | 24.96 | 19 | 28 | 38.14 | 8.7 |
| 14 | 25.42 | 37.6 | 29 | 39.58 | 5 |
| 15 | 26.10 | 11.7 | | | |

### Example 7. Preparation Method for Crystal Form B of Monohydrochloride of Compound of Formula I

The crystal form A was transformed into crystal form B after open storage under acceleration conditions (40 °C/75% RH) for 72 h. The crystal form B is a monohydrate of the monohydrochloride of the compound of formula I.

The crystal form B was characterized by XRPD, DSC, TGA, and PLM.

The locations and intensities of XRPD characteristic peaks are shown in Table 2, and the XRPD pattern is shown in FIG. 5.

DSC shows a first endothermic peak at a peak temperature raised to about 85.87 °C, a second endothermic peak at a peak temperature of about 197.54 °C, and a first exothermic peak at a peak temperature of about 205.68 °C, as shown in FIG. 6.

TGA shows a weight loss of about 3.42% from 21.49 °C to 120 °C and a weight loss of about 0.49% from 179.88 °C to 207.94 °C, as shown in FIG. 7.

The PLM image shows that the sample is a crystal with irregular morphology and a size below 20 µm, as shown in FIG. 8.

In the XRPD pattern of the crystal form B expressed in terms of 2θ angles, the 2θ angles and relative intensities of the diffraction peaks are shown in Table B, wherein the 2θ angles have an error range of ±0.20°:

**Table B. XRPD analysis data for crystal form B**

| Peak number | 2θ [°] | Relative intensity (%) | Peak number | 2θ [°] | Relative intensity (%) |
|---|---|---|---|---|---|
| 1 | 9.48 | 32.4 | 13 | 23.40 | 8.2 |
| 2 | 10.78 | 22.6 | 14 | 24.10 | 13.3 |
| 3 | 12.08 | 7.1 | 15 | 25.41 | 7.8 |
| 4 | 14.68 | 14.5 | 16 | 26.22 | 9 |
| 5 | 15.44 | 27.8 | 17 | 28.16 | 14.4 |
| 6 | 18.10 | 51.2 | 18 | 29.18 | 8 |
| 7 | 19.18 | 20.7 | 19 | 29.58 | 5.5 |
| 8 | 19.80 | 49.9 | 20 | 30.13 | 5.2 |
| 9 | 20.60 | 17.9 | 21 | 30.92 | 24.4 |
| 10 | 21.34 | 12.4 | 22 | 33.02 | 4.6 |
| 11 | 22.10 | 100 | 23 | 35.50 | 8.7 |
| 12 | 22.94 | 9.3 | 24 | 41.28 | 4.8 |

### Example 8. Preparation Method for Crystal Form C of Monohydrochloride of Compound of Formula I

The compound of formula I (3.15 g) was added to a three-necked flask, and methanol (12.6 mL) was added. The mixture was stirred for complete dissolution. A 1.8 N solution of HCl in methanol (3.05 g) was added dropwise at normal temperature. The resulting mixture was stirred for 10 min and then filtered. The filtrate was added to a three-necked flask, and methyl *tert*-butyl ether (78.75 mL) was added dropwise at normal temperature. The mixture was stirred for 2 h and filtered. The filter cake was dried to give the crystal form C (3.06 g, yield: 90%).

The crystal form C is an anhydrate. The crystal form C was characterized by XRPD, DSC, TGA, and PLM.

The locations and intensities of XRPD characteristic peaks are shown in Table 3, and the XRPD pattern is shown in FIG. 9.

DSC shows a first endothermic peak at a peak temperature raised to about 209.93 °C and a first exothermic peak at a peak temperature of about 215.80 °C, as shown in FIG. 10.

TGA shows a weight loss of about 0.29% from 21.62 °C to 120 °C and a weight loss of about 0.52% from 173.94 °C to 216.60 °C, as shown in FIG. 11.

The PLM image shows that the sample is a crystal with irregular morphology and a size below 20 µm, as shown in FIG. 12.

In the XRPD pattern of the crystal form C expressed in terms of 2θ angles, the 2θ angles and relative intensities of the diffraction peaks are shown in Table C:

**Table C. XRPD analysis data for crystal form C**

| Peak number | 2θ [°] | Relative intensity (%) | Peak number | 2θ [°] | Relative intensity (%) |
|---|---|---|---|---|---|
| 1 | 10.00 | 15.7 | 14 | 21.98 | 69.7 |
| 2 | 10.52 | 12.7 | 15 | 22.94 | 36.2 |
| 3 | 11.96 | 21 | 16 | 24.80 | 17.1 |
| 4 | 12.48 | 12.7 | 17 | 25.92 | 40.4 |
| 5 | 13.62 | 13.2 | 18 | 27.46 | 22.7 |
| 6 | 14.74 | 100 | 19 | 29.06 | 27.8 |
| 7 | 15.58 | 10.9 | 20 | 30.30 | 16.3 |
| 8 | 17.80 | 70.2 | 21 | 31.54 | 6.1 |
| 9 | 18.58 | 17.7 | 22 | 32.10 | 13.2 |
| 10 | 18.90 | 14.9 | 23 | 33.48 | 29.3 |
| 11 | 19.58 | 43.8 | 24 | 33.94 | 7.5 |
| 12 | 20.08 | 88.8 | 25 | 36.16 | 6.8 |
| 13 | 21.06 | 20 | 26 | 42.06 | 9.5 |

### Example 9. Preparation Method for Crystal Form D of Monohydrochloride of Compound of Formula I

400 mg of the monohydrochloride of the compound of formula I was taken out, and dichloromethane (8 mL) was added. The mixture was stirred at room temperature for 24 h. The resulting suspension was separated by centrifugation, and the solid was dried in vacuum under reduced pressure at 40 °C, the solid being the crystal form D.

The crystal form D is a mono-dichloromethane solvate (also referred to as monodichloromethane solvate) of the monohydrochloride of the compound of formula I.

The crystal form D was characterized by XRPD, DSC, TGA, and PLM.

The locations and intensities of XRPD characteristic peaks are shown in Table 4, and the XRPD pattern is shown in FIG. 13.

DSC shows a first exothermic peak at a peak temperature raised to about 196.53 °C, as shown in FIG. 14.

TGA shows a weight loss of about 6.31% from 22.07 °C to 120 °C, as shown in FIG. 15.

The PLM image shows that the sample is a crystal with irregular morphology and a size below 10 µm, as shown in FIG. 16.

In the XRPD pattern of the crystal form D expressed in terms of 2θ angles, the 2θ angles and relative intensities of the diffraction peaks are shown in Table D:

**Table D. XRPD analysis data for crystal form D**

| Peak number | 2θ [°] | Relative intensity (%) | Peak number | 2θ [°] | Relative intensity (%) |
|---|---|---|---|---|---|
| 1 | 10.16 | 37.6 | 13 | 23.82 | 43.9 |
| 2 | 11.90 | 25.6 | 14 | 24.94 | 11.8 |
| 3 | 12.60 | 14.6 | 15 | 25.60 | 9.8 |
| 4 | 15.74 | 100 | 16 | 26.24 | 23.3 |
| 5 | 16.58 | 43.5 | 17 | 26.80 | 11.7 |
| 6 | 19.22 | 40.1 | 18 | 27.50 | 12.9 |
| 7 | 19.80 | 13.9 | 19 | 29.36 | 7 |
| 8 | 20.24 | 26.4 | 20 | 29.88 | 13.6 |
| 9 | 21.12 | 9.8 | 21 | 31.00 | 6.6 |
| 10 | 21.98 | 41.1 | 22 | 32.48 | 4.8 |
| 11 | 22.66 | 10.4 | 23 | 36.20 | 6.3 |
| 12 | 23.18 | 14.4 | 24 | 36.76 | 4.4 |

### Example 10. Preparation Method for Crystal Form E of Monohydrochloride of Compound of Formula I

400 mg of the monohydrochloride of the compound of formula I was taken out, and isopropanol (8 mL) was added. The mixture was stirred at room temperature for 72 h. The resulting suspension was separated by centrifugation, and the solid was dried in vacuum under reduced pressure at 40 °C, the solid being the crystal form E.

The crystal form E is a mono-isopropanol solvate of the monohydrochloride of the compound of formula I.

The crystal form E was characterized by XRPD. The XRPD pattern is shown in FIG. 17.

In the XRPD pattern of the crystal form E expressed in terms of 2θ angles, the 2θ angles and relative intensities of the diffraction peaks are shown in Table E:

**Table E. XRPD analysis data for crystal form E**

| Peak number | 2θ [°] | Relative intensity (%) | Peak number | 2θ [°] | Relative intensity (%) |
|---|---|---|---|---|---|
| 1 | 7.20 | 35.5 | 12 | 22.10 | 64.1 |
| 2 | 9.36 | 100 | 13 | 22.70 | 13.1 |
| 3 | 11.32 | 14.4 | 14 | 23.68 | 34.4 |
| 4 | 12.98 | 8 | 15 | 26.04 | 24.2 |
| 5 | 15.22 | 58.7 | 16 | 26.90 | 9.7 |
| 6 | 16.88 | 59 | 17 | 27.86 | 27.3 |
| 7 | 17.90 | 9.5 | 18 | 29.38 | 11.5 |
| 8 | 18.78 | 27.7 | 19 | 30.56 | 10 |
| 9 | 19.18 | 23.1 | 20 | 33.28 | 14.8 |
| 10 | 19.92 | 20 | 21 | 37.54 | 8.4 |
| 11 | 21.10 | 40.3 | | | |

### Example 11. Stability Investigation of Crystal Form C of Monohydrochloride of Compound of Formula I

The crystal form C of the monohydrochloride of the compound of formula I was stored under the conditions of 40 °C/75% RH-closed, 40 °C/75% RH-open, and 60 °C-open and then taken out after 1 month. The stability of the crystal form was investigated.

The chromatographic conditions for the test are shown in Table 14.

Detection method for related substances of the stability sample: about 6 mg of the sample was weighed out and added to a 40 mL clean glass bottle; 10 mL of 50% aqueous acetonitrile solution was added; the sample was dissolved completely by ultrasonication; and 10 µL of the sample was injected for the test of the related substances, as shown in Table 15. The XRPD pattern is shown in FIG. 18, wherein HCl-salt Form 3-initial (hereinafter referred to as initial) represents the XRPD pattern of the crystal form C prepared in Example 8.

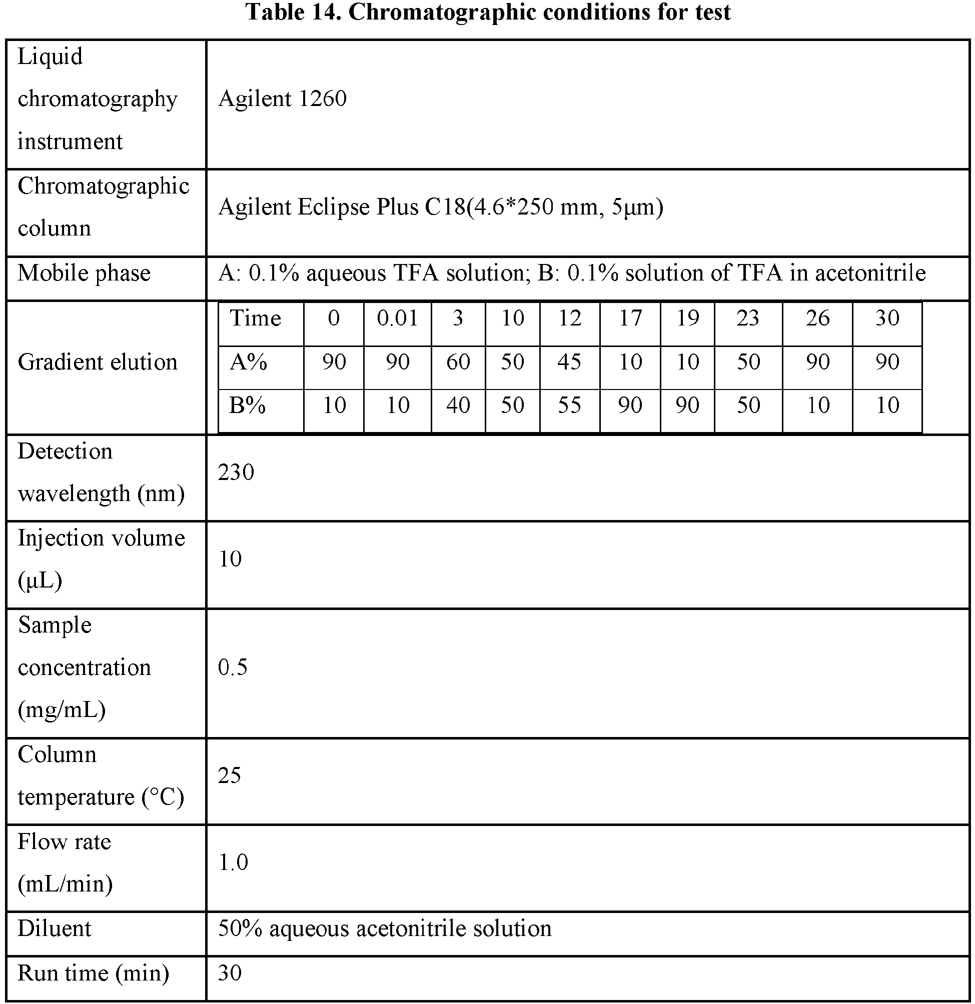

**Table 15. stability test results of crystal form C of monohydrochloride of compound of formula I (1 M)**

| **Salt form** | **Stability condition** | **Appearance** | **Purity%** | **Total impurity%** | **Maximum single impurity%** |
|---|---|---|---|---|---|
| Crystal form C of monohydrochloride of compound of formula I | Initial | White powder | 98.88 | 1.12 | 0.96 |
| Crystal form C of monohydrochloride of compound of formula I | Initial | White powder | 99.17 | 0.83 | 0.65 |
| | 40°C/75%RH-closed-1M | Off-white powder | 99.58 | 0.42 | 0.29 |
| | 40°C75%RH-open-1M | Off-white powder | 99.60 | 0.40 | 0.27 |
| | 60°C-closed-1M | Off-white powder | 99.56 | 0.44 | 0.32 |

| | | | | | |
|---|---|---|---|---|---|
| Note: 1 M represents storage for 1 month. | | | | | |

As can be seen from the above data, the crystal form C of the monohydrochloride of the compound of formula I had good chemical stability after storage at 40 °C/75% RH and at 60 °C for 1 month. In addition, it can be seen from FIG. 18 that the crystal form of the crystal form C of the monohydrochloride of the compound of formula I was not changed.

### Example 12. Effect of High Temperature and High Humidity on Crystal Form C

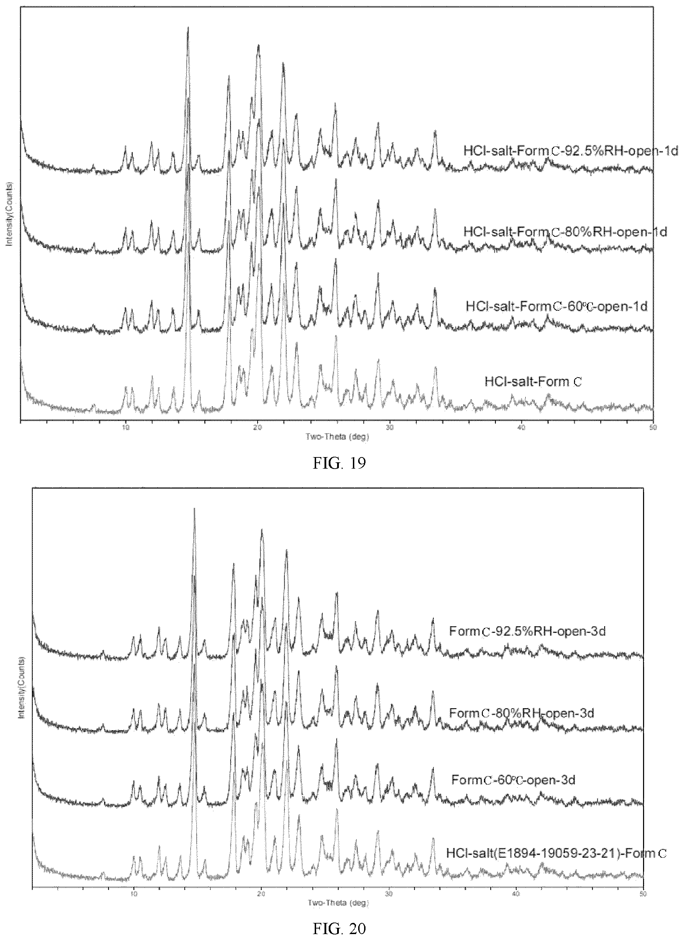

The crystal form C of the monohydrochloride of the compound of formula I was stored in an open flask under the conditions of 60 °C, 80% RH and 92.5% RH, and it was evaluated whether the crystal form was significantly changed. The sample was separately collected on 1d and 3d for XRPD testing. The XRPD patterns are shown in FIGs. 19 and 20.

As can be seen from FIG. 19 and FIG. 20, the crystal form C of the monohydrochloride of the compound of formula I was not changed after exposure to high temperature and high humidity conditions for 1 day and 3 days.

### Example 13. Solubility Tests of Compound of Formula I, Mixture of Crystal Forms A and B, and Crystal Form C

An appropriate amount of a test sample was weighed out and added to a vial; 3 mL of a medium (water, SGF, FaSSIF, FeSSIF, or the like) was added; the mixture was stirred at 37 °C; an appropriate amount of the sample was separately collected at 1 h and 24 h; centrifugation was performed at 12000 rpm for 10 min; the supernatant was diluted with 50% aqueous acetonitrile solution to an appropriate multiple; and the concentration of the supernatant was determined. The chromatographic conditions for the solubility tests are shown in Table 16.

Control and linearity: 10 mg of the compound of formula I was weighed out and added to a 50 mL volumetric flask, 50% aqueous acetonitrile solution was added for dissolution, and the mixture was diluted to volume. Two parts were prepared in parallel. The control was taken and diluted with 50% aqueous acetonitrile solution to 100 µg/mL, 50 µg/mL, and 10 µg/mL, 5 µL of the sample was injected, and a standard curve was plotted.

The test results are shown in Table 17.

**Table 16. Chromatographic conditions for solubility test of monohydrochloride of compound of formula I**

| | |
|---|---|
| Liquid chromatography instrument | Agilent 1260 |
| Chromatographic column | Waters Sunfire C18(4.6*150 mm, 5µm) |
| Mobile phase | A: 0.1% aqueous TFA solution; B: 0.1% solution of TFA in acetonitrile |
| Isocratic elution | A:B=60:40 |
| Detection wavelength (nm) | 230 |
| Injection volume (µL) | 5 |
| Sample concentration (mg/mL) | 0.5 |
| Column temperature (°C) | 40 |
| Flow rate (mL/min) | 1.0 |
| Diluent | 50% aqueous acetonitrile solution |
| Run time (min) | 30 |

**Table 17. Solubility results of compound of formula I, mixture of crystal forms A and B, and crystal form C (37 °C)**

| **Sample** | **Medium** | **Sample amount (mg)** | **Medium volume (mL)** | **Solubility at 1 h (mg/mL)** | **Solubility at 24 h (mg/mL)** | **Sample appearance-24 h** | **Final pH** |
|---|---|---|---|---|---|---|---|
| Compound of formula I | Water | 31.89 | 3 | 0.37 | 0.31 | White suspension | 8.73 |
| | SGF | 15.73 | 3 | 5.51 | 5.48 | Pale purple solution | 1.74 |
| | FassIF | 16.35 | 3 | 0.22 | 0.19 | White suspension | 6.44 |
| | FessIF | 16.45 | 3 | 0.37 | 0.35 | White suspension | 5.03 |
| Mixture of crystal form A and crystal form B | Water | 33.71 | 3 | 11.74 | 11.14 | Light grey suspension | 2.49 |
| | SGF | 16.29 | 3 | 5.90 | 5.97 | Pale purple solution | 1.52 |
| | FassIF | 16.73 | 3 | 0.64 | 0.39 | White suspension | 3.83 |
| | FessIF | 15.82 | 3 | 0.74 | 0.44 | White suspension | 4.85 |
| Crystal form C | Water | 31.34 | 3 | 8.32 | 7.54 | White suspension | 2.49 |
| | SGF | 17.36 | 3 | 0.94 | 0.79 | White suspension | 1.26 |
| | FassIF | 15.94 | 3 | 1.16 | 0.88 | White suspension | 3.27 |
| | FessIF | 15.35 | 3 | 0.64 | 0.42 | White suspension | 4.83 |
| Crystal form C | pH6.8 | 10.45 | 5 | 0.56(1h) | N\A | White suspension | 6.65 |
| | pH7.4 | 10.30 | 5 | 0.45(1h) | N\A | White suspension | 7.23 |
| | pH6.8 | 10.45 | 5 | N\A | 0.21(48h) | White suspension | 6.82 |
| | pH7.4 | 10.30 | 5 | N\A | 0.24(48h) | White suspension | 7.42 |

| | | | | | | | |
|---|---|---|---|---|---|---|---|
| *Note: the pH value in pH 6.8 and pH 7.4 was adjusted with a sodium hydroxide solution so that the final pH was close to the initial value.* | | | | | | | |

As can be seen from the experimental results described above, the crystal form C had reduced solubility in water compared to the mixture of crystal forms A and B.

The exemplary embodiments of the present disclosure have been described above. However, the protection scope of the present application is not limited to the exemplary embodiments described above. Any modification, equivalent replacement, improvement, and the like made by those skilled in the art without departing from the spirit and principle of the present disclosure shall fall within the protection scope as defined by the claims of the present application.

## Claims

1. A pharmaceutically acceptable salt of a compound of formula I: 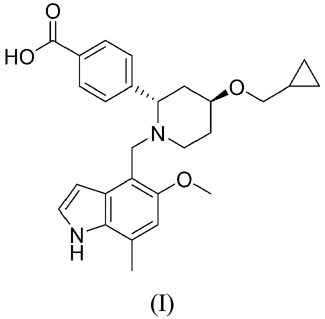
wherein the pharmaceutically acceptable salt is a salt formed by the compound of formula I and an acid or a base;
wherein the acid is selected from one of hydrochloric acid, hydrofluoric acid, hydrobromic acid, hydroiodic acid, sulfuric acid, pyrosulfuric acid, phosphoric acid, nitric acid, formic acid, acetic acid, acetoacetic acid, pyruvic acid, trifluoroacetic acid, propionic acid, butyric acid, caproic acid, enanthic acid, undecanoic acid, lauric acid, benzoic acid, salicylic acid, 2-(4-hydroxybenzoyl)benzoic acid, camphoric acid, cinnamic acid, cyclopentanepropionic acid, digluconic acid, 3-hydroxy-2-naphthoic acid, nicotinic acid, pamoic acid, pectinic acid, persulfuric acid, 3-phenylpropionic acid, picric acid, pivalic acid, 2-hydroxyethanesulfonic acid, itaconic acid, sulfamic acid, trifluoromethanesulfonic acid, dodecylsulfuric acid, ethanesulfonic acid, benzenesulfonic acid, *p*-toluenesulfonic acid, methanesulfonic acid, 2-naphthalenesulfonic acid, naphthalenedisulfonic acid, camphorsulfonic acid, citric acid, L-tartaric acid, stearic acid, lactic acid, oxalic acid, malonic acid, succinic acid, malic acid, adipic acid, alginic acid, maleic acid, fumaric acid, D-gluconic acid, mandelic acid, ascorbic acid, glucoheptonic acid, glycerophosphoric acid, aspartic acid, sulfosalicylic acid, hemisulfuric acid, or thiocyanic acid; preferably selected from one of hydrochloric acid, hydrobromic acid, sulfuric acid, phosphoric acid, nitric acid, methanesulfonic acid, *p*-toluenesulfonic acid, fumaric acid, maleic acid, citric acid, L-tartaric acid, oxalic acid, formic acid, acetic acid, trifluoroacetic acid, lauric acid, benzoic acid, and benzenesulfonic acid;
the base is selected from an alkali metal hydroxide or an alkaline-earth metal hydroxide, preferably selected from sodium hydroxide or potassium hydroxide;
preferably, the pharmaceutically acceptable salt of the compound of formula I is selected from one of hydrochloride, sulfate, phosphate, methanesulfonate, *p*-toluenesulfonate, fumarate, maleate, citrate, L-tartrate, and oxalate thereof;
more preferably, the pharmaceutically acceptable salt of the compound of formula I is a salt formed by the compound of formula I and hydrochloric acid; more preferably, the pharmaceutically acceptable salt of the compound of formula I is monohydrochloride formed by the compound of formula I and hydrochloric acid.

2. A preparation method for the pharmaceutically acceptable salt of the compound of formula I according to claim 1, wherein the preparation method comprises: reacting the compound of formula I with the acid or the base to give the pharmaceutically acceptable salt of the compound of formula I;
preferably, the preparation method comprises: reacting the compound of formula I with the acid or the base in a solvent to give the pharmaceutically acceptable salt of the compound of formula I;
preferably, the solvent is selected from an alcohol, a ketone, an ester, an ether, a combination of two or more of the solvents, or a mixture of the solvent described above with water or a mixture of the combination described above with water;
preferably, the alcohol is selected from an alcohol having 1 to 8 carbon atoms, e.g., methanol, ethanol, *n*-propanol, isopropanol, *n*-butanol, neopentyl alcohol, or a combination of two or more thereof; the ketone may be selected from a ketone having 3 to 10 carbon atoms, e.g., acetone, butanone, pentanone, methyl ethyl ketone, 4-methyl-2-pentanone, or a combination of two or more thereof; the ester may be selected from an organic carboxylic ester, e.g., methyl formate, ethyl acetate, isobutyl formate, isopropyl acetate, or a combination of two or more thereof; and the ether may be a linear or branched alkyl ether or a cyclic ether compound, e.g., methyl *tert*-butyl ether, tetrahydrofuran, 2-methyl-tetrahydrofuran, or a combination of two or more thereof;
preferably, the compound of formula I and the acid or the base are in a molar ratio of 1:0.8 to 1:1.5; the acid or the base, independently of each other, has the definitions described in claim 1.

3. A single crystal of the monohydrochloride of the compound of formula I according to claim 1, wherein the single crystal has the following unit cell parameters:
an orthorhombic crystal system with a space group of *P*2₁2₁2₁;
*a* = 9.4704 (18) Å;
*b =* 15.324 (4) Å;
*c* = 17.437 (4) Å;
*V =* 2530.5 (10) A³; and
Z=4.

4. A crystal form A of the monohydrochloride of the compound of formula I according to claim 1, wherein the crystal form A has characteristic peaks at 2θ angles of 9.66±0.20°, 16.08±0.20°, and 23.46±0.20° by X-ray powder diffraction using Cu-Kα radiation;
preferably, the crystal form A has characteristic peaks at 2θ angles of 9.66±0.20°, 16.08±0.20°, 18.10±0.20°, 21.30±0.20°, and 21.68±0.20° by X-ray powder diffraction using Cu-Kα radiation;
preferably, the crystal form A has characteristic peaks at 2θ angles of 9.66±0.20°, 11.62±0.20°, 16.08±0.20°, 18.10±0.20°, 21.30±0.20°, 21.68±0.20°, 23.40±0.20°, and 25.42±0.20° by X-ray powder diffraction using Cu-Kα radiation;
preferably, the crystal form A has characteristic peaks at 2θ angles of 9.66±0.20°, 11.62±0.20°, 16.08±0.20°, 16.84±0.20°, 18.10±0.20°, 19.64±0.20°, 21.30±0.20°, 21.68±0.20°, 23.40±0.20°, 24.96±0.20°, and 25.42±0.20° by X-ray powder diffraction using Cu-Kα radiation;
preferably, the crystal form A has the following characteristic peaks at 2θ angles by X-ray powder diffraction using Cu-Kα radiation, wherein the 2θ angles have an error range of ±0.20°:
| Peak number | 2θ [ ° ] | Peak number | 2θ [ ° ] |
|---|---|---|---|
| 1 | 8.02 | 16 | 26.78 |
| 2 | 9.66 | 17 | 27.66 |
| 3 | 11.62 | 18 | 28.44 |
| 4 | 12.78 | 19 | 28.70 |
| 5 | 14.32 | 20 | 29.04 |
| 6 | 16.08 | 21 | 29.70 |
| 7 | 16.84 | 22 | 30.14 |
| 8 | 18.10 | 23 | 32.38 |
| 9 | 19.64 | 24 | 33.82 |
| 10 | 21.30 | 25 | 35.41 |
| 11 | 21.68 | 26 | 36.89 |
| 12 | 23.46 | 27 | 37.92 |
| 13 | 24.96 | 28 | 38.14 |
| 14 | 25.42 | 29 | 39.58 |
| 15 | 26.10 | | |
preferably, the crystal form A has an X-ray powder diffraction pattern substantially as shown in FIG. 1;
preferably, the crystal form A is an anhydrate of the monohydrochloride of the compound of formula I;
preferably, the crystal form A has a DSC profile substantially as shown in FIG. 2;
preferably, the crystal form A has a TGA profile substantially as shown in FIG. 3;
preferably, the crystal form A is a crystal with irregular morphology; preferably, the crystal form A has a particle size of no more than 20 µm;
preferably, the crystal form A has a PLM image substantially as shown in FIG. 4.

5. A crystal form B of the monohydrochloride of the compound of formula I according to claim 1, wherein the crystal form B has characteristic peaks at 2θ angles of 18.10±0.20°, 19.80±0.20°, and 22.10±0.20° by X-ray powder diffraction using Cu-Kα radiation;
preferably, the crystal form B has characteristic peaks at 2θ angles of 9.48±0.20°, 15.44±0.20°, 18.10±0.20°, 19.80±0.20°, 22.10±0.20°, and 30.92±0.20° by X-ray powder diffraction using Cu-Kα radiation;
preferably, the crystal form B has characteristic peaks at 2θ angles of 9.48±0.20°, 10.78±0.20°, 15.44±0.20°, 18.10±0.20°, 19.18±0.20°, 19.80±0.20°, 22.10±0.20°, and 30.92±0.20° by X-ray powder diffraction using Cu-Kα radiation;
preferably, the crystal form B has the following characteristic peaks at 2θ angles by X-ray powder diffraction using Cu-Kα radiation, wherein the 2θ angles have an error range of ±0.20°:
| Peak number | 2θ [ ° ] | Peak number | 2θ [ ° ] |
|---|---|---|---|
| 1 | 9.48 | 13 | 23.40 |
| 2 | 10.78 | 14 | 24.10 |
| 3 | 12.08 | 15 | 25.41 |
| 4 | 14.68 | 16 | 26.22 |
| 5 | 15.44 | 17 | 28.16 |
| 6 | 18.10 | 18 | 29.18 |
| 7 | 19.18 | 19 | 29.58 |
| 8 | 19.80 | 20 | 30.13 |
| 9 | 20.60 | 21 | 30.92 |
| 10 | 21.34 | 22 | 33.02 |
| 11 | 22.10 | 23 | 35.50 |
| 12 | 22.94 | 24 | 41.28 |
preferably, the crystal form B has an X-ray powder diffraction pattern substantially as shown in FIG. 5;
preferably, the crystal form B is a hydrate of the monohydrochloride of the compound of formula I, such as a monohydrate of the monohydrochloride of the compound of formula I;
preferably, the crystal form B has a DSC profile substantially as shown in FIG. 6;
preferably, the crystal form B has a TGA profile substantially as shown in FIG. 7;
preferably, the crystal form B is a crystal with irregular morphology; preferably, the crystal form B has a particle size of no more than 20 µm;
preferably, the crystal form B has a PLM image substantially as shown in FIG. 8.

6. A crystal form C of the monohydrochloride of the compound of formula I according to claim 1, wherein the crystal form C has characteristic peaks at 2θ angles of 14.74±0.20°, 17.80±0.20°, 20.08±0.20°, and 21.98±0.20° by X-ray powder diffraction using Cu-Kα radiation;
preferably, the crystal form C has characteristic peaks at 2θ angles of 14.74±0.20°, 17.80±0.20°, 19.58±0.20°, 20.08±0.20°, 21.98±0.20°, 22.94±0.20°, and 25.92±0.20° by X-ray powder diffraction using Cu-Kα radiation;
preferably, the crystal form C has characteristic peaks at 2θ angles of 14.74±0.20°, 17.80±0.20°, 19.58±0.20°, 20.08±0.20°, 21.98±0.20°, 22.94±0.20°, 25.92±0.20°, and 33.48±0.20° by X-ray powder diffraction using Cu-Kα radiation;
preferably, the crystal form C has the following characteristic peaks at 2θ angles by X-ray powder diffraction using Cu-Kα radiation, wherein the 2θ angles have an error range of ±0.20°:
| Peak number | 2θ [ ° ] | Peak number | 2θ [ ° ] |
|---|---|---|---|
| 1 | 10.00 | 14 | 21.98 |
| 2 | 10.52 | 15 | 22.94 |
| 3 | 11.96 | 16 | 24.80 |
| 4 | 12.48 | 17 | 25.92 |
| 5 | 13.62 | 18 | 27.46 |
| 6 | 14.74 | 19 | 29.06 |
| 7 | 15.58 | 20 | 30.30 |
| 8 | 17.80 | 21 | 31.54 |
| 9 | 18.58 | 22 | 32.10 |
| 10 | 18.90 | 23 | 33.48 |
| 11 | 19.58 | 24 | 33.94 |
| 12 | 20.08 | 25 | 36.16 |
| 13 | 21.06 | 26 | 42.06 |
preferably, the crystal form C has an X-ray powder diffraction pattern substantially as shown in FIG. 9;
preferably, the crystal form C is an anhydrate of the monohydrochloride of the compound of formula I;
preferably, the crystal form C has a DSC profile substantially as shown in FIG. 10;
preferably, the crystal form C has a TGA profile substantially as shown in FIG. 11;
preferably, the crystal form C is a crystal with irregular morphology; preferably, the crystal form C has a particle size of no more than 20 µm;
preferably, the crystal form C has a PLM image substantially as shown in FIG. 12.

7. A crystal form D of the monohydrochloride of the compound of formula I according to claim 1, wherein the crystal form D has characteristic peaks at 2θ angles of 15.74±0.20°, 16.58±0.20°, 21.98±0.20°, and 23.82±0.20° by X-ray powder diffraction using Cu-Kα radiation;
preferably, the crystal form D has characteristic peaks at 2θ angles of 10.16±0.20°, 11.90±0.20°, 15.74±0.20°, 16.58±0.20°, 19.22±0.20°, 20.24±0.20°, 21.98±0.20°, and 23.82±0.20° by X-ray powder diffraction using Cu-Kα radiation;
preferably, the crystal form D has characteristic peaks at 2θ angles of 10.16±0.20°, 11.90±0.20°, 12.60±0.20°, 15.74±0.20°, 16.58±0.20°, 19.22±0.20°, 19.80±0.20°, 21.98±0.20°, 22.66±0.20°, 23.18±0.20°, 23.82±0.20°, 24.94±0.20°, 26.24±0.20°, 26.80±0.20°, and 27.50±0.20° by X-ray powder diffraction using Cu-Kα radiation;
preferably, the crystal form D has the following characteristic peaks at 2θ angles by X-ray powder diffraction using Cu-Kα radiation, wherein the 2θ angles have an error range of ±0.20°:
| Peak number | 2θ [ ° ] | Peak number | 2θ [ ° ] |
|---|---|---|---|
| 1 | 10.16 | 13 | 23.82 |
| 2 | 11.90 | 14 | 24.94 |
| 3 | 12.60 | 15 | 25.60 |
| 4 | 15.74 | 16 | 26.24 |
| 5 | 16.58 | 17 | 26.80 |
| 6 | 19.22 | 18 | 27.50 |
| 7 | 19.80 | 19 | 29.36 |
| 8 | 20.24 | 20 | 29.88 |
| 9 | 21.12 | 21 | 31.00 |
| 10 | 21.98 | 22 | 32.48 |
| 11 | 22.66 | 23 | 36.20 |
| 12 | 23.18 | 24 | 36.76 |
preferably, the crystal form D has an X-ray powder diffraction pattern substantially as shown in FIG. 13;
preferably, the crystal form D is a solvate of the monohydrochloride of the compound of formula I, for example, a dichloromethane solvate of the monohydrochloride of the compound of formula I, such as a mono-dichloromethane solvate of the monohydrochloride of the compound of formula I;
preferably, the crystal form D has a DSC profile substantially as shown in FIG. 14;
preferably, the crystal form D has a TGA profile substantially as shown in FIG. 15;
preferably, the crystal form D is a crystal with irregular morphology; preferably, the crystal form D has a particle size of no more than 10 µm;
preferably, the crystal form D has a PLM image substantially as shown in FIG. 16.

8. A crystal form E of the monohydrochloride of the compound of formula I according to claim 1, wherein the crystal form E has characteristic peaks at 2θ angles of 9.36±0.20°, 15.22±0.20°, 16.88±0.20°, and 22.10±0.20° by X-ray powder diffraction using Cu-Kα radiation;
preferably, the crystal form E has characteristic peaks at 2θ angles of 7.20±0.20°, 9.36±0.20°, 15.22±0.20°, 16.88±0.20°, 21.10±0.20°, 22.10±0.20°, and 23.68±0.20° by X-ray powder diffraction using Cu-Kα radiation;
preferably, the crystal form E has characteristic peaks at 2θ angles of 7.20±0.20°, 9.36±0.20°, 15.22±0.20°, 16.88±0.20°, 18.78±0.20°, 21.10±0.20°, 22.10±0.20°, 23.68±0.20°, 26.04±0.20°, and 27.86±0.20° by X-ray powder diffraction using Cu-Kα radiation;
preferably, the crystal form E has the following characteristic peaks at 2θ angles by X-ray powder diffraction using Cu-Kα radiation, wherein the 2θ angles have an error range of ±0.20°:
| Peak number | 2θ [ ° ] | Peak number | 2θ [ ° ] |
|---|---|---|---|
| 1 | 7.20 | 12 | 22.10 |
| 2 | 9.36 | 13 | 22.70 |
| 3 | 11.32 | 14 | 23.68 |
| 4 | 12.98 | 15 | 26.04 |
| 5 | 15.22 | 16 | 26.90 |
| 6 | 16.88 | 17 | 27.86 |
| 7 | 17.90 | 18 | 29.38 |
| 8 | 18.78 | 19 | 30.56 |
| 9 | 19.18 | 20 | 33.28 |
| 10 | 19.92 | 21 | 37.54 |
| 11 | 21.10 | | |
preferably, the crystal form E has an X-ray powder diffraction pattern substantially as shown in FIG. 17;
preferably, the crystal form E is a solvate of the monohydrochloride of the compound of formula I, such as a mono-isopropanol solvate of the monohydrochloride of the compound of formula I.

9. A preparation method for the crystal form A, the crystal form B, the crystal form C, the crystal form D, or the crystal form E according to any one of claims 4-8, wherein:
the preparation method for the crystal form A comprises one of methods selected from the following:
method 1, comprising: dissolving the compound of formula I in an alcohol solvent, adding a solution of HCl in the alcohol solvent to form a salt, and then adding an n-alkane for crystallization to give the crystal form A;
wherein preferably, the alcohol solvent is selected from ethanol and/or isopropanol;
preferably, the n-alkane is selected from n-hexane and/or n-heptane;
preferably, the compound of formula I, the alcohol solvent, and the n-alkane are in a mass-to-volume ratio of 1 g:(10-30) mL:(10-30) mL;
preferably, the solution of HCl in the alcohol solvent is at a concentration of 1-3 mol/L; and
method 2, comprising: heating and stirring the monohydrochloride of the compound of formula I in an alcohol solvent and an n-alkane until it is completely dissolved, and then crystallizing to give the crystal form A;
wherein preferably, the alcohol solvent is selected from ethanol and/or isopropanol;
preferably, the n-alkane is selected from n-hexane and/or n-heptane;
preferably, the monohydrochloride of the compound of formula I, the alcohol solvent, and the n-alkane are in a mass-to-volume ratio of 1 g:(10-30) mL:(10-30) mL;
preferably, the heating temperature is 45-75 °C;
the preparation method for the crystal form B comprises: putting the crystal form A according to claim 4 under a high humidity condition to give the crystal form B;
preferably, the high humidity condition has a temperature of 30-50 °C and a humidity of 60%-98%;
preferably, the high humidity condition has a temperature of 40 °C and a humidity of 75%-95%;
the preparation method for the crystal form C comprises: dissolving the compound of formula I in an alcohol solvent, adding a solution of HCl in the alcohol solvent to form a salt, and then adding an ether solvent or an ester solvent for crystallization to give the crystal form C;
preferably, the alcohol solvent is selected from methanol, ethanol, or isopropanol;
preferably, the ether solvent is selected from methyl ether, diethyl ether, propyl ether, or methyl *tert*-butyl ether;
preferably, the ester solvent is selected from ethyl acetate or isopropyl acetate;
preferably, the compound of formula I, the alcohol solvent, and the ether solvent or ester solvent are in a mass-to-volume ratio of 1 g:(2-8) mL:(20-40) mL;
preferably, the solution of HCl in the alcohol solvent is at a concentration of 1-3 mol/L; the compound of formula I and the solution of HCl in the alcohol solvent are in a mass ratio of 1 g:(0.5-1.5) g;
the preparation method for the crystal form D comprises: suspending and stirring the monohydrochloride of the compound of formula I in a haloalkane at room temperature for crystallization to give the crystal form D;
preferably, the haloalkane is selected from dichloromethane, trichloromethane, or tetrachloromethane;
preferably, the monohydrochloride of the compound of formula I and the haloalkane are in a mass-to-volume ratio of 1 g:(15-35) mL;
the preparation method for the crystal form E comprises: suspending and stirring the monohydrochloride of the compound of formula I in an alcohol solvent at room temperature for crystallization to give the crystal form E;
preferably, the alcohol solvent is selected from methanol, ethanol, or isopropanol;
preferably, the monohydrochloride of the compound of formula I and the alcohol solvent are in a mass-to-volume ratio of 1 g:(15-35) mL.

10. A pharmaceutical composition comprising at least one of the pharmaceutically acceptable salt of the compound of formula I, the crystal form A, the crystal form B, the crystal form C, the crystal form D, and the crystal form E according to any one of claims 1-8, and optionally a pharmaceutically acceptable auxiliary material;
preferably, the pharmaceutical composition is in the form of a preparation.

11. Use of at least one of the pharmaceutically acceptable salt of the compound of formula I, the crystal form A, the crystal form B, the crystal form C, the crystal form D, and the crystal form E according to any one of claims 1-8 or the pharmaceutical composition according to claim 10 for the manufacturing of a medicament for preventing and/or treating a complement factor B-mediated disease or disorder; wherein
preferably, the complement factor B-mediated disease or disorder is selected from at least one of the following: paroxysmal nocturnal hemoglobinuria (PNH), primary glomerulonephritis (IgAN), membranous nephropathy (MN), C3 glomerulonephritis (C3G), age-related macular degeneration (AMD), geographic atrophy (GA), atypical hemolytic uremic syndrome (aHUS), hemolytic uremic syndrome (HUS), diabetic retinopathy (DR), hemodialysis complications, hemolytic anemia or hemodialysis, neuromyelitis optica (NMO), arthritis, rheumatoid arthritis, liver-related inflammations, dermatomyositis and amyotrophic lateral sclerosis, myasthenia gravis (MG), respiratory diseases and cardiovascular diseases.
